# EUROPEAN PATENT APPLICATION

(11) **EP 3 620 519 A1**
(43) Date of publication of application: **11.03.2020**
(21) Application number: 18192614.8
(22) Date of filing: 04.09.2018
(51) Int. Cl.: C12N 15/113, A61K 31/712, A61K 31/7125, A61K 35/20

(54) **USE OF ISOLATED MILK EXTRACELLULAR VESICLES FOR DELIVERING OLIGONUCLEOTIDES ORALLY**

(71) Applicant: F. Hoffmann-La Roche AG, 4070 Basel (CH)
(72) Inventor: The designation of the inventor has not yet been filed
(74) Representative: Hansen, Marianne Rudolph

(57) **Abstract**

The present invention provides an isolated milk extracellular vesicle carrying a therapeutic single-stranded antisense oligonucleotide with at least one non-natural modification, such milk extracellular vesicles are also termed loaded milk extracellular vesicles. The loaded milk extracellular vesicles of the invention are for use as a medicament, and are particularly useful when administered orally, delivering the therapeutic single-stranded antisense oligonucleotide to one or more of the target tissues selected from the group consisting of the central nervous system, spleen, liver and T-cells. Oral administration of the loaded isolated milk extracellular vesicle of the invention is beneficial in the treatment of a range of diseases including central nervous system diseases (such as brain diseases), spleen diseases, liver diseases and diseases involving the T-cells.

SEQUENCE LISTING

<110> F. Hoffmann-La Roche AG

<120> Use of isolated milk extracellular vesicles for delivering
oligonucleotides orally

<130> P34980-EP

<160> 2

<170> BiSSAP 1.3.6

<210> 1
<211> 16
<212> DNA
<213> Artificial Sequence

<220>
<223> oligonucleotide

<400> 1
gagttacttg ccaact 16

<210> 2
<211> 18
<212> DNA
<213> Artificial Sequence

<220>
<223> oligonucleotide

<400> 2
cagagttact tgccaact 18

## Description

### FIELD OF INVENTION

The present invention provides an isolated milk extracellular vesicle carrying a therapeutic single-stranded antisense oligonucleotide with at least one non-natural modification, such milk extracellular vesicles are also termed loaded milk extracellular vesicles. The loaded milk extracellular vesicles of the invention are for use as a medicament, and are particularly useful when administered orally, delivering the therapeutic single-stranded antisense oligonucleotide to one or more of the target tissues selected from the group consisting of the central nervous system, spleen, liver and T-cells. Oral administration of the loaded isolated milk extracellular vesicle of the invention is beneficial in the treatment of a range of diseases including central nervous system diseases (such as brain diseases), spleen diseases, liver diseases and diseases involving the T-cells.

### BACKGROUND

The delivery of drugs to specific cells and tissues is influenced by many factors, such as the stability of the drug, the metabolism of the drug before reaching the target tissue, the ability of the drug to cross cell membranes, tight junctions between cells or the blood-brain barrier.

Exosomes are extracellular vesicles secreted by all types of cells. They are thought to play an important role in cell-to-cell communication, which is achieved through the transfer of exosome cargos such as micro RNAs (miRNAs) from donor cells to recipient cells. Recently, exosomes from many different sources have been proposed as carriers for pharmaceutical agents.

For example, it has been shown that exosomes derived from bovine milk have can serve as a drug delivery vehicle. Munagala describes the isolation of exosomes from bovine milk and the encapsulation of a chemotherapeutic agent into the isolated exosomes. Drug-loaded exosomes showed significantly higher efficacy compared to free drug in cell culture studies and against lung tumor xenografts *in vivo.* Further, Munagala analyzed the distribution of exosomes after oral gavage and intravenous administration. According to Munagala, the tissue distribution of exosomes labeled with the lipophilic fluorescent dye DiR on the surface (not loaded with therapeutic agent) was somewhat uniform within the liver, lung, kidney, pancreas, spleen, ovaries, colon and brain with the gavage route (Munagala et al., Cancer Lett. 2016 February 1; 371(1): 48-61).

WO 2014/134132 discloses a composition comprising an effective amount of a therapeutic agent encapsulated by a milk-derived microvesicle. The authors of the scientific publication Munagala et al. (see above) are named as inventors for the international application. The experimental data provided in WO 2014/134132 essentially correspond to the data in the scientific publication.

Manca analyzes the distribution of milk exosomes comprising synthetic miRNAs after oral administration. According to Manca, distinct miRNA cargos have unique tissue distribution patterns. Administration of bovine milk exosomes failed to rescue Drosha homozygous knockout mice (Manca et al., Scientific Reports (2018) 8:11321).

Shandilaya discloses that siRNAs encapsulated in milk exosomes are resistant to digestion and cross the intestinal barrier *in vitro* (J. Agric. Food Chem. 2017, 65, 9506-9513).

WO2018/102397 discloses that milk exosomes comprising therapeutic agents could be used in disease treatment. The milk exosomes can be administered orally and used for delivering otherwise poorly orally available drugs. In the examples section, the loading of siRNA into milk exosomes and their physico-chemical properties are analyzed, there are no *in vitro* or *in vivo* data. The document further mentions that an antibody, a hormone, a factor, a cofactor, a metabolic enzyme, an immunoregulatory enzyme, an interferon, an interleukin, a gastrointestinal enzyme, an enzyme or factor implicated in hemostasis, a growth regulatory enzyme, a vaccine, an antithrombolytic, a toxin, single-stranded or double-stranded DNA, a mRNA, a non-coding RNA (ncRNA), an antisense RNA, a LNA, or a morpholino oligonucleotide could be encapsulated in milk exosomes. However, without data it remains unknown whether exosomes can be loaded with such drugs and whether their biodistribution and toxicity profile would make the suitable as medicaments. In addition, the document is silent on the biodistribution of exosomes and payload after oral administration.

Single-stranded modified antisense oligonucleotides are generally administered systemically via intravenous or subcutaneous administration, when delivered by this route liver and kidney are the primary target tissues. To reach targets in the CNS local delivery such as for example intrathecal or intracerebroventricular administration. These administration forms generally do not require any delivery vehicles to result in an effect on the target, although certain conjugates have shown a benefit in terms of potency. siRNAs on the contrary generally do need delivery vehicles to be effective. There are some examples of oral delivery of single-stranded modified antisense oligonucleotides using permeation enhancer to loosen the tight junctions in the gut, see for example Tillman et al 2008 J Pharm Sci 97 p.225 and WO2015/188194. Roberts et al 2006 Molecular Therapy 14 p. 471 also show oral delivery of a LNA modified splice switching oligonucleotide when administered at 50mg/kg for 4 days.

Oral delivery of therapeutic antisense oligonucleotides is not commonly used and has to our knowledge never been shown to result in target modulation in the CNS, spleen or T-cells.

### OBJECTIVE OF THE INVENTION

The inventors have shown in the studies underlying the present invention that oral administration of milk extracellular vesicles comprising a modified single-stranded antisense oligonucleotide, such as an antisense oligonucleotide comprising at least one modified internucleoside linkage, allows for an efficient modulation of target nucleic acid expression in the central nervous system (e.g. the brain), the spleen, the liver and/or in T-cells (see Examples). Therefore, milk extracellular vesicles comprising a modified single-stranded antisense oligonucleotide, when administered orally, are capable of delivering modified oligonucleotides to one or more of the target tissues selected from the group consisting of the central nervous system, spleen, liver and T-cells resulting in modulation of a target nucleic acid in the tissue. In addition, no toxic side effects were observed after oral administration. The findings of the present invention are particularly useful in the treatment of a range of diseases, such as diseases which are associated with abnormal expression, such as increased expression, reduced expression or undesired suppression, slice-switching errors or mutational errors, of a target nucleic in the central nervous system (e.g. the brain), the spleen, the liver and/or in T-cells.

### STATEMENT OF THE INVENTION

The invention provides an isolated milk extracellular vesicle carrying a modified single-stranded antisense oligonucleotide for use as a medicament, wherein said isolated milk extracellular vesicle is administered orally.

The invention provides an isolated milk extracellular vesicle carrying a modified single-stranded antisense oligonucleotide for use as a medicament, wherein said isolated milk extracellular vesicle is administered orally, wherein said modified single-stranded antisense oligonucleotide is delivered to one or more of the target tissues selected from the group consisting of the central nervous system, spleen, liver and T-cells.

The invention provides an isolated milk extracellular vesicle carrying a modified single-stranded antisense oligonucleotide for use as a medicament, wherein said isolated milk extracellular vesicle is administered orally, wherein expression of a target nucleic acid in one or more of the target tissues selected from the group consisting of the central nervous system, spleen, liver and T-cells is modulated.

The modified single-stranded antisense oligonucleotide typically has a length of 7 to 35 nucleotides, such as 7 to 30 nucleotides.

The modified single-stranded antisense oligonucleotide has a modified backbone, such as one or more modified internucleoside linkages and/or one or more modified sugar moieties. Accordingly, the expressions "modified single-stranded antisense oligonucleotide" and "single-stranded antisense oligonucleotide comprising at least one backbone modification" are used interchangeably herein.

One aspect of the invention is an isolated milk extracellular vesicle carrying a single-stranded antisense oligonucleotide of 7 to 35 nucleotides with at least one backbone modification for use as a medicament, wherein said isolated milk extracellular vesicle is administered orally, and wherein said single-stranded antisense oligonucleotide is delivered to one or more of the target tissues selected from the group consisting of the central nervous system, spleen, liver and T-cells

In an embodiment, the modified single-stranded antisense oligonucleotide has at least one modified internucleoside linkage, such as at least 75% modified internucleoside linkages.

In an embodiment, the modified single-stranded antisense oligonucleotide has at least one modified nucleoside, such as at least 3 2' sugar modified nucleosides.

In an embodiment, the modified single-stranded antisense oligonucleotide is a single-stranded morpholino antisense oligonucleotide.

In an embodiment, the modified single-stranded antisense oligonucleotide is single-stranded peptide nucleic acids (PNA).

### BRIEF DESCRIPTION OF FIGURES

**Figure 1****.** SDS-Page gel of milk, milk serum, crude extracellular vesicles (BMEVs), density gradient centrifugation fractions, and purified BMEVs of fractions 7 and 8. Denatured samples were separated on a 4-15% gradient TGX acrylamide gel. Proteins were visualized after UV activation. The black box on the SDS gel at fraction 6 to 8 indicates the bands representing the purified EV's (exosomes).
**Figure 2****.** Western blot analysis of extracellular vesicle (BMEV) markers. Denatured samples were separated on a 4-15% gradient TGX acrylamide gel. Proteins were then blotted on a PVDF membrane and TSG101 and CD9 were stained and visualized using HRP-conjugated secondary antibodies.
**Figure 3****.** Transmission electron micrograph of extracellular vesicles (BMEVs). BMEVs were adsorbed on Formvar coated copper grids and were negatively stained using 2% uranyl acetate. Images were then acquired on a JEM-1400 plus transmission electron microscope.
Scale bar: 100 nm.

### DEFINITIONS

### Isolated

The term "isolated", when applied in connection with the milk extracellular vesicle, refers to a milk extracellular vesicle that has been isolated from its native environment, i.e. from milk.

In context of the present invention the isolated milk extracellular vesicle has been further modified by encapsulating a modified single-stranded antisense oligonucleotide. Accordingly, the isolated milk extracellular vesicle carrying a modified single-stranded antisense oligonucleotide does not occur naturally.

### Extracellular vesicles/ Milk extracellular vesicles

Extracellular vesicles, sometimes also referred to as exosomes, are naturally existing microvesicles that are small assemblies of lipid/protein containing particles of about 30 to 500 nm in size often with a cargo of naturally occurring miRNAs. They are secreted by many types of *in vitro* cell cultures and *in vivo* cells, and are commonly found *in vivo* in body fluids. Extracellular vesicles are formed by a variety of processes such as the release of apoptotic bodies, the budding of extracellular vesicles directly from the cytoplasmic membranes of cells, and exocytosis from multivesicular bodies.

The extracellular vesicles to be applied in accordance with the present invention are milk extracellular vesicles, and thus have been isolated from milk such as from cow milk, sheep milk or goat milk. The isolation of extracellular vesicles from milk is well known in the art, one example is described in the Materials and Method section.

Preferably, the isolated milk extracellular vesicles have a diameter, a Zeta potential and/or a polydispersity index (PDI) as indicated here.

The PDI shall be lower than 0.3, such as lower than 0.25, such as lower than 0.2. Alternatively, the isolated milk extracellular vesicles as set forth herein may have a polydispersity index (PDI) in the range of 0 to 0.4, such as in the range of 0.05 to 0.3, such as in the range of 0.1 to 0.25, such as in the range of 0.1 to 0.25. The polydispersity index is a measure of the vesicle size distribution within a population of vesicles. The PDI can be calculated by dividing the diameter of the vesicles (see above) by the square of the standard deviation. In an embodiment, the isolated milk extracellular vesicles as set forth herein may have a polydispersity index (PDI) in the range of 0 to 0.4.

The isolated milk extracellular extracellular vesicles may have a diameter in the range of 30 to 500 nm, such as a diameter is in the range of 70 to 400 nm, or in the range of 100 to 300 nm, or in the range for 150 to 250 nm, or in the range from 150 to 200 nm. As used herein, the term "diameter" refers to the mean hydrodynamic diameter (e.g. of vesicles in a population of vesicles), such as the mean hydrodynamic diameter determined by dynamic light scattering (also referred to as Photon Correlation Spectroscopy), e.g. at a laser wavelength of 685 nm. In particular, the "diameter" refers to the mean hydrodynamic diameter as determined as described in the Materials and Method section.

Further, the isolated milk extracellular vesicles as may have a Zeta potential in the range of 0 to -50 (minus 50) mV, such as in the range of -5 to -40 mV, such as in the range of -15 to -35 mV, such as in the range from -20 to -30 mV. The Zeta potential is the electrokinetic potential in colloidal dispersions. As used herein the term "Zeta potential" refers to the Zeta potential as determined as described in the Materials and Method section.

### Oligonucleotide

The term "oligonucleotide" as used herein is defined as it is generally understood by the skilled person as a molecule comprising two or more covalently linked nucleosides. Such covalently bound nucleosides may also be referred to as nucleic acid molecules or oligomers. Oligonucleotides are commonly made in the laboratory by solid-phase chemical synthesis followed by purification. When referring to a sequence of the oligonucleotide, reference is made to the sequence or order of nucleobase moieties, or modifications thereof, of the covalently linked nucleotides or nucleosides. The oligonucleotide of the invention is man-made, and is chemically synthesized, and is typically purified or isolated.

### Antisense oligonucleotide

The term "Antisense oligonucleotide" as used herein is defined as oligonucleotides capable of modulating expression of a target nucleic acid by hybridizing to a target nucleic acid, in particular to a contiguous sequence on a target nucleic acid. The antisense oligonucleotides are not essentially double stranded and are therefore not siRNAs or shRNAs. Further, antisense oligonucleotides are not miRNAs, particularly not naturally occurring miRNAs. The antisense oligonucleotides are single stranded. It is understood that single stranded oligonucleotides can form hairpins or intermolecular duplex structures (duplex between two molecules of the same oligonucleotide), as long as the degree of intra or inter self-complementarity is less than 50% across of the full length of the oligonucleotide.

### Contiguous Nucleotide Sequence

The term "contiguous nucleotide sequence" refers to the region of the oligonucleotide which is complementary to the target nucleic acid. The term is used interchangeably herein with the term "contiguous nucleobase sequence" and the term "oligonucleotide motif sequence". In some embodiments all the nucleotides of the oligonucleotide constitute the contiguous nucleotide sequence. In some embodiments the oligonucleotide comprises the contiguous nucleotide sequence which hybridized to the target nucleic acid, such as a F-G-F' gapmer region, and may optionally comprise further nucleotide(s), for example a nucleotide linker region which may be used to attach a functional group to the contiguous nucleotide sequence. The nucleotide linker region may or may not be complementary to the target nucleic acid. Adventurously, the contiguous nucleotide sequence is 100% complementary to the target nucleic acid.

### Nucleotides

Nucleotides are the building blocks of oligonucleotides and polynucleotides, and for the purposes include both naturally occurring and non-naturally occurring nucleotides. In nature, nucleotides, such as DNA and RNA nucleotides comprise a ribose sugar moiety, a nucleobase moiety and one or more phosphate groups (which is absent in nucleosides). Nucleosides and nucleotides may also interchangeably be referred to as "units" or "monomers".

### Nucleobase

The term nucleobase includes the purine (e.g. adenine and guanine) and pyrimidine (e.g. uracil, thymine and cytosine) moiety present in nucleosides and nucleotides which form hydrogen bonds in nucleic acid hybridization. In the context of the present invention the term nucleobase also encompasses modified nucleobases which may differ from naturally occurring nucleobases, but are functional during nucleic acid hybridization. In this context "nucleobase" refers to both naturally occurring nucleobases such as adenine, guanine, cytosine, thymidine, uracil, xanthine and hypoxanthine, as well as non-naturally occurring variants. Such variants are for example described in Hirao et al (2012) Accounts of Chemical Research vol 45 page 2055 and Bergstrom (2009) Current Protocols in Nucleic Acid Chemistry Suppl. 37 1.4.1.

The nucleobase moieties may be indicated by the letter code for each corresponding nucleobase, e.g. A, T, G, C or U, wherein each letter may optionally include modified nucleobases of equivalent function. For example, in the exemplified oligonucleotides, the nucleobase moieties are selected from A, T, G, C, and 5-methyl cytosine. Optionally, for LNA containing oligonucleotides, 5-methyl cytosine LNA nucleosides may be used.

### Modified internucleoside linkages

The term "modified internucleoside linkage" is defined as generally understood by the skilled person as linkages other than phosphodiester (PO) linkages, that covalently couples two nucleosides together. The oligonucleotides of the invention may therefore comprise modified internucleoside linkages. In some embodiments, the modified internucleoside linkage increases the nuclease resistance of the oligonucleotide compared to a phosphodiester linkage. For naturally occurring oligonucleotides, the internucleoside linkage includes phosphate groups creating a phosphodiester bond between adjacent nucleosides. Modified internucleoside linkages are particularly useful in stabilizing oligonucleotides for *in vivo* use, and may serve to protect against nuclease cleavage at regions of DNA or RNA nucleosides in the oligonucleotide of the invention, for example within the gap region of a gapmer oligonucleotide, as well as in regions of modified nucleosides, such as region F and F'.

In an embodiment, the oligonucleotide comprises one or more internucleoside linkages modified from the natural phosphodiester, such one or more modified internucleoside linkages that is for example more resistant to nuclease attack. Nuclease resistance may be determined by incubating the oligonucleotide in blood serum or by using a nuclease resistance assay (e.g. snake venom phosphodiesterase (SVPD)), both are well known in the art. Internucleoside linkages which are capable of enhancing the nuclease resistance of an oligonucleotide are referred to as nuclease resistant internucleoside linkages.

In an embodiment, the modified single stranded antisense oligonucleotide comprises at least one modified internucleoside linkage. In some embodiments at least 50% of the internucleoside linkages in the oligonucleotide as referred to herein, or contiguous nucleotide sequence thereof, are modified internucleoside linkages, such as at least 60%, such as at least 70%, such as at least 80% or such as at least 90% of the internucleoside linkages in the oligonucleotide, or contiguous nucleotide sequence thereof, are modified internucleoside linkages. In some embodiments all of the internucleoside linkages of the oligonucleotide, or contiguous nucleotide sequence thereof, are modified internucleoside linkages.

In some embodiments all of the internucleoside linkages of the oligonucleotide, or contiguous nucleotide sequence thereof, are nuclease resistant internucleoside linkages. It will be recognized that, in some embodiments the nucleosides which link the oligonucleotide of the invention to a non-nucleotide functional group, such as a conjugate, may be phosphodiester.

Preferably, the modified internucleoside linkage is a phosphorothioate internucleoside linkage, a diphosphorothioate internucleoside linkage, or a boranophosphate internucleoside linkage.

A preferred modified internucleoside linkage is a phosphorothioate internucleoside linkage. Phosphorothioate internucleoside linkages are particularly useful due to nuclease resistance, beneficial pharmacokinetics and ease of manufacture. In some embodiments at least 50% of the internucleoside linkages in the oligonucleotide, or contiguous nucleotide sequence thereof, are phosphorothioate linkages, such as at least 60%, such as at least 70%, such as at least 80% or such as at least 90% of the internucleoside linkages in the oligonucleotide, or contiguous nucleotide sequence thereof, are phosphorothioate linkages. In some embodiments all of the internucleoside linkages of the oligonucleotide, or contiguous nucleotide sequence thereof, are phosphorothioate linkages.

Nuclease resistant linkages, such as phosphorothioate linkages, are particularly useful in oligonucleotide regions capable of recruiting nuclease when forming a duplex with the target nucleic acid, such as region G for gapmers. Phosphorothioate linkages may, however, also be useful in non-nuclease recruiting regions and/or affinity enhancing regions such as regions F and F' for gapmers, or in mixmers and totalmers. Gapmer oligonucleotides may, in some embodiments comprise one or more phosphodiester linkages in region F or F', or both region F and F', which the internucleoside linkage in region G may be fully phosphorothioate. Advantageously, all the internucleoside linkages in the contiguous nucleotide sequence of the oligonucleotide are phosphorothioate linkages.

It is recognized that, as disclosed in EP2 742 135, antisense oligonucleotide may comprise other internucleoside linkages (other than phosphodiester and phosphorothioate), for example alkyl phosphonate / methyl phosphonate internucleoside, which according to EP2 742 135 may for example be tolerated in an otherwise DNA phosphorothioate gap region.

### Modified nucleoside

The term "modified nucleoside" or "nucleoside modification" as used herein refers to nucleosides modified as compared to the equivalent DNA or RNA nucleoside by the introduction of one or more chemical modifications of the moieties forming the backbone of the oligonucleotide, such as modifications to the sugar moiety or substitution of the sugar moiety with for example morpholino moieties or peptide nucleic acid (PNA) moieties. The term modified nucleoside may also be used herein interchangeably with the term "nucleoside analogue" or modified "units" or modified "monomers". Nucleosides with an unmodified DNA or RNA sugar moiety are termed DNA or RNA nucleosides herein. Nucleosides with modifications in the base region of the DNA or RNA nucleoside are still generally termed DNA or RNA if they allow Watson Crick base pairing.

Below are illustrations of two linked PNA units and two linked morpholino units. Examples of 2' sugar modified nucleosides can be found in a separate definition thereof.

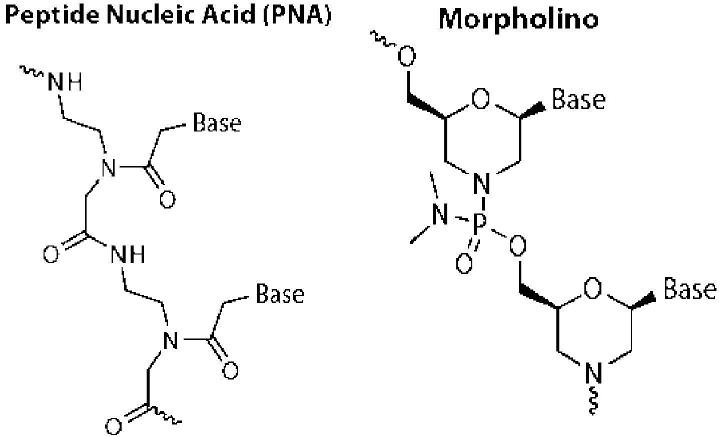

### 2' sugar modified nucleosides.

A 2' sugar modified nucleoside is a nucleoside which has a substituent other than H or -OH at the 2' position (2' substituted nucleoside) or comprises a 2' linked biradicle capable of forming a bridge between the 2' carbon and a second carbon in the ribose ring, such as LNA (2' - 4' biradicle bridged) nucleosides.

Indeed, much focus has been spent on developing 2' substituted nucleosides, and numerous 2' substituted nucleosides have been found to have beneficial properties when incorporated into oligonucleotides. For example, the 2' modified sugar may provide enhanced binding affinity and/or increased nuclease resistance to the oligonucleotide. Examples of 2' substituted modified nucleosides are 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleoside. For further examples, please see e.g. Freier & Altmann; Nucl. Acid Res., 1997, 25, 4429-4443 and Uhlmann; Curr. Opinion in Drug Development, 2000, 3(2), 293-213, and Deleavey and Damha, Chemistry and Biology 2012, 19, 937. Below are illustrations of some 2' substituted modified nucleosides.

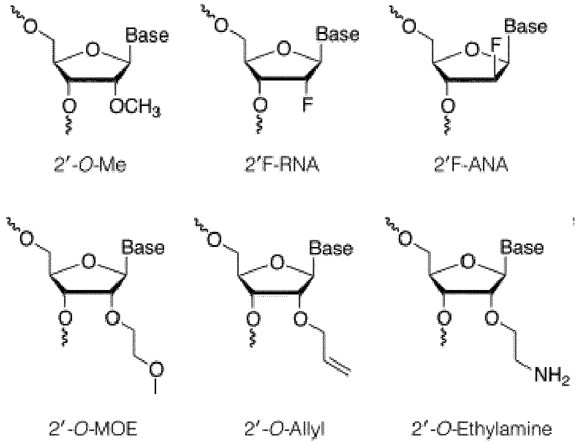

In relation to the present invention 2' substituted does not include 2' bridged molecules like LNA.

### Modified antisense oligonucleotide

As used in herein a modified antisense oligonucleotide is an oligonucleotide where the oligonucleotide backbone has been modified compared to the natural occurring RNA or DNA oligonucleotides. The oligonucleotide backbone refers to the internucleotide linkage and/or the sugar moiety, but generally not to modifications in the base moieties.

Modification of the antisense oligonucleotide backbone is commonly used for therapeutic oligonucleotides to increase stability, in particular towards nucleases or to increase affinity towards the target nucleic acid.

Stability increasing modifications are for example modified internucleoside linkages, some sugar modifications as well as peptide backbones. Modifications increasing affinity are for example sugar modifications.

The modified single stranded antisense oligonucleotide as referred to herein comprises one or more modified nucleosides and/or modified internucleoside linkages. Modified oligonucleotides can comprise a mixture of 2' sugar modified nucleotides and DNA or RNA nucleosides, for example in a gapmer or mixmer design these are also sometimes termed "chimeric" oligonucleotides. PNA and morpholino oligonucleotides are generally composed of the same backbone, such that they are composed of PNA moieties or morpholino moieties only.

The modified oligonucleotide may in some embodiments comprise or consist of the contiguous nucleotide sequence of the oligonucleotide which is complementary to the target nucleic acid, such as the gapmer, mixmer or totalmer region, and further 5' and/or 3' nucleosides which may not be fully complementary to the target nucleic acid. Such additional terminal nucleosides can for example be 2 to 4 phosphodiester linked DNA nucleosides and serve as a biocleavable linker between the contiguous nucleotide sequence complementary to the target nucleic acid and for example a conjugate moiety.

The modified single-stranded antisense oligonucleotide typically has a length of 7 to 35 nucleotides. In an embodiment, the modified single-stranded antisense oligonucleotide has a length of 7 to 30 nucleotides. In another embodiment, the modified single-stranded antisense oligonucleotide has a length of 10 to 30 nucleotides. In another embodiment, the modified single-stranded antisense oligonucleotide has a length of 14 to 30 nucleotides. In another embodiment, the modified single-stranded antisense oligonucleotide has a length of 14 to 20 nucleotides. In another embodiment, the modified single-stranded antisense oligonucleotide has a length of 7 to 14 nucleotides.

The term antisense oligonucleotide in the singular form "an antisense oligonucleotide" or "a modified antisense oligonucleotide" refers to a population of oligonucleotides which share a common nucleobase sequence and pattern of chemical modifications and are typically derived from a common manufacturing process.

### Locked Nucleic Acids (LNA)

A "LNA nucleoside" is a 2'- modified nucleoside which comprises a biradical linking the C2' and C4' of the ribose sugar ring of said nucleoside (also referred to as a "2'- 4' bridge"), which restricts or locks the conformation of the ribose ring. These nucleosides are also termed bridged nucleic acid or bicyclic nucleic acid (BNA) in the literature. The locking of the conformation of the ribose is associated with an enhanced affinity of hybridization (duplex stabilization) when the LNA is incorporated into an oligonucleotide for a complementary RNA or DNA molecule. This can be routinely determined by measuring the melting temperature of the oligonucleotide/complement duplex.

Non limiting, exemplary LNA nucleosides are disclosed in WO 99/014226, WO 00/66604, WO 98/039352 , WO 2004/046160, WO 00/047599, WO 2007/134181, WO 2010/077578, WO 2010/036698, WO 2007/090071, WO 2009/006478, WO 2011/156202, WO 2008/154401, WO 2009/067647, WO 2008/150729, Morita et al., Bioorganic & Med.Chem. Lett. 12, 73-76, Seth et al. J. Org. Chem. 2010, Vol 75(5) pp. 1569-81, and Mitsuoka et al., Nucleic Acids Research 2009, 37(4), 1225-1238, and Wan and Seth, J. Medical Chemistry 2016, 59, 9645-9667.

Further non limiting, exemplary LNA nucleosides are disclosed in Scheme 1.

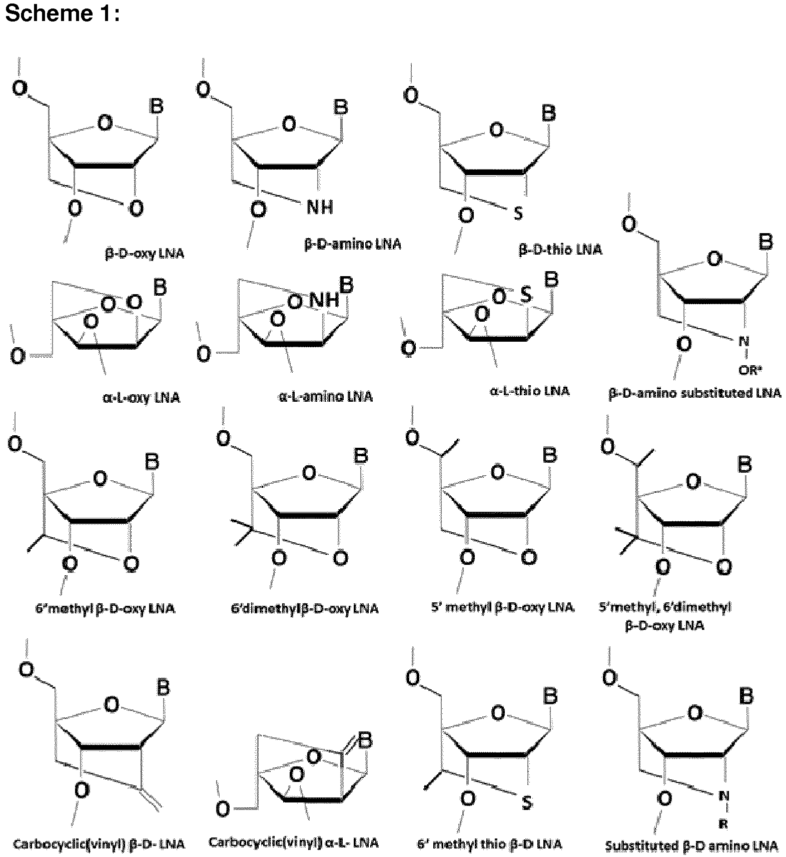

Particular LNA nucleosides are beta-D-oxy-LNA, 6'-methyl-beta-D-oxy LNA such as (S)-6'-methyl-beta-D-oxy-LNA (ScET) and ENA.

A particularly advantageous LNA is beta-D-oxy-LNA.

### Gapmer

The modified single stranded antisense oligonucleotide comprised by the isolated extracellular milk exosome as referred to herein may be a gapmer, also termed gapmer oligonucleotide or gapmer designs. The antisense gapmers are commonly used to inhibit a target nucleic acid via RNase H mediated degradation. A gapmer oligonucleotide comprises at least three distinct structural regions a 5'-flank, a gap and a 3'-flank, F-G-F' in the '5 -> 3' orientation. The "gap" region (G) comprises a stretch of contiguous DNA nucleotides which enable the oligonucleotide to recruit RNase H, such as from 5 - 16 contiguous DNA nucleotides. The gap region is flanked by a 5' flanking region (F) comprising one or more sugar modified nucleosides, advantageously high affinity 2' sugar modified nucleosides, and by a 3' flanking region (F') comprising one or more sugar modified nucleosides, advantageously high affinity 2' sugar modified nucleosides. The flanking regions independently consist of 1 - 8 contiguous nucleotides with a sugar modified nucleoside defining each end. The one or more sugar modified nucleosides in region F and F' enhance the affinity of the oligonucleotide for the target nucleic acid (*i.e.* are affinity enhancing sugar modified nucleosides). In some embodiments, the one or more sugar modified nucleosides in region F and F' are 2' sugar modified nucleosides, such as high affinity 2' sugar modifications, such as independently selected from LNA and 2'-MOE.

In a gapmer design, the 5' and 3' most nucleosides of the gap region are DNA nucleosides, such as 70%, 75%, 80%, 85%, 90% or 100%. A DNA is always positioned adjacent to a sugar modified nucleoside of the 5' (F) or 3' (F') region respectively. The Gap region may in some instances contain modified nucleosides which do not prevent RNase H cleavage, for example, alpha-L-LNA, C4' alkylated DNA (as described in WO/2009/090182 and Vester et al., Bioorg. Med. Chem. Lett. 18 (2008) 2296 - 2300, both incorporated herein by reference), arabinose derived nucleosides like ANA and 2'F-ANA (Mangos et al. 2003 J. AM. CHEM. SOC. 125, 654-661), UNA (unlocked nucleic acid) (as described in Fluiter et al., Mol. Biosyst., 2009, 10, 1039 incorporated herein by reference. The flanks may further be defined by having at least one sugar modified nucleoside at the end most distant from the gap region, i.e. at the 5' end of the 5' flank and at the 3' end of the 3' flank.

Regions F-G-F' form a contiguous nucleotide sequence. Antisense oligonucleotides of the invention, or the contiguous nucleotide sequence thereof, may comprise a gapmer region of formula F-G-F'.

The overall length of the gapmer design F-G-F' may be, for example 12 to 32 nucleosides, such as 13 to 24, such as 14 to 22 nucleosides, Such as from 14 to 20, such as 16 to18 nucleosides. By way of example, the gapmer oligonucleotide of the present invention can be represented by the following formulae:

F₁₋₈-G₅₋₁₆-F'₁₋₈,

such as

F₁₋₈-G₇₋₁₆-F'₂₋₈

with the proviso that the overall length of the gapmer regions F-G-F' is at least 12, such as at least 14 nucleotides in length.

### LNA Gapmer

The modified single stranded antisense oligonucleotide comprised by the isolated extra-cellular milk exosome as referred to herein may be a LNA gapmer. An LNA gapmer is a gapmer wherein either one or both of region F and F' comprises or consists of LNA nucleosides. A beta-D-oxy gapmer is a gapmer wherein either one or both of region F and F' comprises or consists of beta-D-oxy LNA nucleosides.

In a classic LNA gapmer both flanks consist of LNA nucleosides. However alternating flank LNA gapmers have also been described, where one or both of the flanking regions comprise both LNA and DNA nucleosides (see for example WO2016/127002). In such alternating designs, the flanking region F or F', or both F and F' comprise at least three nucleosides, wherein the 5' and 3' most nucleosides of the F and/or F' region are LNA nucleosides.

In some embodiments the LNA gapmer is of formula: [LNA]₁₋₅-[G]₆₋₁₆-[LNA]₁₋₅, wherein G is the RNase recruiting gap region. LNA gapmers with a 3-10-3 (LNA-DNA-LNA) design has been widely used in the prior art.

### MOE Gapmers

The modified single stranded antisense oligonucleotide comprised by the isolated extra-cellular milk exosome as referred to herein may be a MOE gapmer. A MOE gapmers is a gapmer wherein regions F and F' consist of MOE nucleosides. In some embodiments the MOE gapmer is of design [MOE]₁₋₈-[G]₅₋₁₆-[MOE]₁₋₈, such as [MOE]₂₋₇-[G]₆₋₁₄-[MOE]₂₋₇, such as [MOE]₃₋₆-[G]₈₋₁₂-[MOE]₃₋₆, wherein G is the RNase recruiting gap region. MOE gapmers with a 5-10-5 design (MOE-DNA-MOE) have been widely used in the art.

### Mixed Wing Gapmer

The modified single stranded antisense oligonucleotide comprised by the isolated extra-cellular milk exosome as referred to herein may be a mixed wing gapmer. A mixed wing gapmer is an LNA gapmer wherein one or both of region F and F' comprise a 2' substituted nucleoside, such as a 2' substituted nucleoside independently selected from the group consisting of 2'-O-alkyl-RNA units, 2'-O-methyl-RNA, 2'-amino-DNA units, 2'-fluoro-DNA units, 2'-alkoxy-RNA, MOE units, arabino nucleic acid (ANA) units and 2'-fluoro-ANA units. In some embodiments at least one of region F and F', or both region F and F' comprise at least one LNA nucleoside, the remaining nucleosides of region F and F' are independently selected from the group consisting of MOE and LNA. In some mixed wing embodiments, one or both of region F and F' may further comprise one or more DNA nucleosides.

Mixed wing gapmer designs are disclosed in WO2008/049085 and WO2012/109395, both of which are hereby incorporated by reference.

### Totalmer

The modified single stranded antisense oligonucleotide comprised by the isolated extra-cellular milk exosome as referred to herein may be a totalmer. A totalmer is a single stranded modified antisense oligonucleotide, or contiguous nucleotide sequence thereof, which does not comprise DNA or RNA nucleosides, and generally only comprises one type of nucleoside analogues. The contiguous nucleotide sequence of a totalmer oligonucleotide is generally between 7 and 16 nucleotides, such as between 8 and 12 2' sugar modified nucleosides which can be selected from the group consisting of 2'-O-alkyl-RNA, 2'-O-methyl-RNA, 2'-alkoxy-RNA, 2'-O-methoxyethyl-RNA, 2'-amino-DNA, 2'-fluoro-DNA, arabino nucleic acid (ANA), 2'-fluoro-ANA and LNA (locked nucleic acid) nucleosides.

Generally short totalmers (below 10 nucleotides in length) are composed of 100% LNA units. For longer totalmers one or more of the nucleoside units may be selected from the non-LNA nucleotide analogues such as 2' sugar substituted nucleosides referred to herein.

In some embodiments the totalmer consist or comprises of a contiguous nucleotide sequence which consists only of LNA units.

PNA and morpholino oligonucleotides can also be considered as totalmers.

Totalmer designs have shown to be effective as therapeutic oligonucleotides, particularly when targeting microRNA (antimiRs) or as splice switching oligonucleotides (SSOs).

### Mixmer

The modified single stranded antisense oligonucleotide comprised by the isolated extra-cellular milk exosome as referred to herein may be a mixmer. A mixmer is a single stranded modified antisense oligonucleotide, or contiguous nucleotide sequences thereof, where as opposed to gapmers, there is no contiguous sequence of more than 5 naturally occurring DNA nucleosides, and therefore it does not recruit RNase H.

Generally, the mixmer comprises or consists of a contiguous nucleotide sequence of repeating pattern of one type of nucleoside analogue, such as a 2' sugar modified nucleoside, and a naturally occurring nucleoside, such as DNA. This could for example be a repeating pattern of LNA nucleosides and DNA nucleosides starting and ending with an LNA in the 5' end and 3' end. The mixmer may however also combine different types of nucleoside analogues, such that the repeating pattern for instance is every second or every third nucleoside is a nucleoside analogue, such as LNA, and the remaining nucleosides are naturally occurring nucleosides, such as DNA, or a 2'sugar substituted nucleoside analogue such as 2'MOE of 2'fluoro analogues or other 2' sugar substituted nucleosides mentioned herein. The mixmer generally has a nucleoside analogue (independently selected) at the 5' or 3' termini. Examples of mixmers are described in WO2007/027894, WO2007/112754, WO2007/112754 (antimiRs) and WO2008/131807 (SSOs). The distribution pattern of nucleoside analogues and DNA is subject to optimization depending on the oligonucleotide sequence.

Mixmer designs are highly effective as therapeutic oligonucleotides, particularly when targeting microRNA (antimiRs), microRNA binding sites on mRNAs (Blockmirs) or as splice-switching oligomers (SSOs).

### antimiR

The modified single stranded antisense oligonucleotide comprised by the isolated extra-cellular milk exosome as referred to herein may be an antimiR. An antimiR, also known as an anti-miRNA oligonucleotide, is an antisense oligonucleotide capable of controlling the *in vivo* activity of a microRNA (miRNA). miRNAs are complementary sequences (approximately 22 bp) to mRNA and are involved in the cleavage of RNA or the suppression of the translation. The antimiRs primarily function by sequestering the mature miRNA in competition with the cellular target mRNAs thereby leading to functional inhibition of the miRNA and de-repression of the direct targets (see for example WO2009/043353 and Stenvang et al 2012 Silence 3:1). AntimiRs are designed with an increased binding affinity for the microRNA and increased nuclease resistance to improve the their ability of prevent binding of the microRNA to the target mRNA. AntimiRs are generally designed as totalmers or mixmers not inducing RNase H cleavage.

### Blockmir

The modified single stranded antisense oligonucleotide comprised by the isolated extra-cellular milk exosome as referred to herein may be a blockmir. A blockmir, also known as an antagomir, is an antisense oligonucleotide that hat prevent other molecules from binding to a desired site on an mRNA molecule. This can for example be the microRNA binding sites on a mRNA thereby preventing its regulation by the miRNA's. So where an antimiR targets the miRNA directly the blockmir only targets a specific mRNA preventings it interaction with for example miRNAs. Blockmirs are generally designed as totalmers or mixmers not inducing RNase H cleavage.

### Splice-switching antisense oligonucleotide (SSO)

The modified single stranded antisense oligonucleotide comprised by the isolated extra-cellular milk exosome as referred to herein may be a splice-switching oligonucleotide. A splice-switching oligonucleotide (SSOs) is an antisense oligonucleotide that base-pair with a pre-mRNA and disrupt the normal splicing repertoire of the transcript by blocking the RNA-RNA base-pairing or protein-RNA binding interactions that occur between components of the splicing machinery and the pre-mRNA. Modulation of splicing is particularly valuable in cases of disease caused by mutations that lead to disruption of normal splicing or when interfering with the normal splicing process of a gene transcript may be therapeutic (see for example Havens and Hastings 2016 Nucleic Acid Research 44:6549). Currently there are two approved SSO molecules on the market, Exondys 51 (morpholino SSO) to treat Duchenne muscular dystrophy and Spinraza (MOE totalmer SSO) to treat spinal muscular atrophy. SSOs are generally designed as totalmers or mixmers not inducing RNase H cleavage.

### Conjugate

The term conjugate as used herein refers to an oligonucleotide which is covalently linked to a non-nucleotide moiety (conjugate moiety or region C or third region).

Conjugation of the single stranded antisense oligonucleotide to a lipid may improve the encapsulation of the single stranded antisense oligonucleotide into the milk extracellular vesicle by increasing the hydrophobicity of the oligonucleotide.

In one embodiment the single stranded antisense oligonucleotide which is to be encapsulated into a milk extracellular vesicle is conjugated at the 5' or 3' terminal with a lipophilic conjugate moiety. The lipohphilic conjugate moiety may be selected from the group consisting of sterols, stanols, steroids, polycyclic aromatic groups, aliphatic groups, lipids, phospholipids, lipophilic alcohols, fatty acids and fatty esters.

In some embodiments, the conjugate moiety is or comprises a lipid, a phospholipid e.g. di-hexadecyl-rac-glycerol or triethylammonium 1,2-di-o-hexadecyl-rac-glycero-3-h-phosphonate or a lipophilic alcohol, a palmityl moiety, a cationic lipid, a neutral lipids, sphingolipids, and fatty acids such as stearic, oleic, elaidic, linoleic, linoleaidic, linolenic, and myristic acids. In some embodiments the fatty acid comprises a C4 - C30 saturated or unsaturated alkyl chain. The alkyl chain may be linear or branched.

Lipophilic conjugate moieties include, for example, sterols stanols, and steroids and related compounds such as cholesterol (U.S. Pat. No. 4,958,013 and Letsinger et al., Proc. Natl. Acad. Sci. USA, 1989, 86, 6553), thiocholesterol (Oberhauser et al, Nucl Acids Res., 1992, 20, 533), lanosterol, coprostanol, stigmasterol, ergosterol, calciferol, cholic acid, deoxycholic acid, estrone, estradiol, estratriol, progesterone, stilbestrol, testosterone, tocopherol, androsterone, deoxycorticosterone, cortisone, 17-hydroxycorticosterone, their derivatives, and the like. In some embodiments, the conjugate may be selected from the group consisting of cholesterol, thiocholesterol, hexylamino-carbonyl-oxycholesterol, lanosterol, coprostanol, stigmasterol, ergosterol, calciferol, cholic acid, deoxycholic acid, estrone, estradiol, estratriol, progesterone, stilbestrol, testosterone, androsterone, deoxycorticosterone, cortisone, and 17-hydroxycorticosterone. In some embodiments the conjugate moiety comprises tocopherol. In some embodiments, the conjugate moiety comprises cholesterol.

Other lipophilic conjugate moieties include aliphatic groups, such as, for example, straight chain, branched, and cyclic alkyls, alkenyls, and alkynyls. The aliphatic groups can have, for example, 5 to about 50, 6 to about 50, 8 to about 50, or 10 to about 50 carbon atoms. Example aliphatic groups include e.g., dodecandiol or undecyl residues, dodecyl, hexadecyl, heptadecyl, octadecyl, nonadecyl, terpenes, bornyl, adamantyl, derivatives thereof and the like. In some embodiments, one or more carbon atoms in the aliphatic group can be replaced by a heteroatom such as O, S, or N (e.g., geranyloxyhexyl). Further suitable lipophilic conjugate moieties include aliphatic derivatives of glycerols such as alkylglycerols, bis(alkyl)glycerols, tris(alkyl)glycerols, monoglycerides, diglycerides, and triglycerides. In some embodiments, the lipophilic conjugate is di-hexyldecyl-rac-glycerol or 1,2-di-O- hexyldecyl-rac-glycerol (Manoharan et al., Tetrahedron Lett., 1995, 36, 3651; Shea, et al., Nuc. Acids Res., 1990, 18, 3777) or phosphonates thereof. Saturated and unsaturated fatty functionalities, such as, for example, fatty acids, fatty alcohols, fatty esters, and fatty amines, can also serve as lipophilic conjugate moieties. In some embodiments, the fatty functionalities can contain from about 6 carbons to about 30 or about 8 to about 22 carbons. Example fatty acids include, capric, caprylic, lauric, palmitic, myristic, stearic, oleic, linoleic, linolenic, arachidonic, eicosenoic acids and the like.

In further embodiments, lipophilic conjugate moieties can be polycyclic aromatic groups having from 6 to about 50, 10 to about 50, or 14 to about 40 carbon atoms. Example polycyclic aromatic groups include pyrenes, purines, acridines, xanthenes, fluorenes, phenanthrenes, anthracenes, quinolines, isoquinolines, naphthalenes, derivatives thereof and the like. Other suitable lipophilic conjugate moieties include menthols, trityls (e.g., dimethoxytrityl (DMT)), phenoxazines, lipoic acid, phospholipids, ethers, thioethers (e.g., hexyl-S-tritylthiol), derivatives thereof and the like. Preparation of lipophilic conjugates of oligonucleotides are well-described in the art, such as in, for example, Saison-Behmoaras et al, EMBO J., 1991, 10, 1111; Kabanov et al., FEBSLett., 1990, 259, 327; Svinarchuk et al, Biochimie, 1993, 75, 49; (Mishra et al., Biochim. Biophys. Acta, 1995, 1264, 229, and Manoharan et al., Tetrahedron Lett., 1995, 36, 3651.

### Biocleavable nucleotide linkers

The single-stranded antisense oligonucleotide which is to be encapsulated into a milk extracellular vesicle may in some embodiments comprise or consist of the contiguous nucleotide sequence of the oligonucleotide which is complementary to the target nucleic acid, such as the gapmer F-G-F', the totalmer or the mixmer regions and may further comprise additional 5' and/or 3' nucleosides. The further 5' and/or 3' nucleosides may or may not be fully complementary to the target nucleic acid, such further 5' and/or 3' nucleosides may be referred to as region D' and D" herein.

The addition of region D' or D" may be used for the purpose of joining the contiguous nucleotide sequence, such as the contiguous nucleotide sequence of the gapmer, totalmer or mixmer, to a conjugate moiety or another functional group in a manner that allows removal of the conjugate from the contiguous nucleoside sequence once it has reached its target tissue. To form a biocleavable linker between the contiguous nucleotide sequence and the conjugate moiety the additional nucleosides (region D' or D") are linked with a nuclease liable linkage such as a phosphodiester internucleoside linkages. Region D' or D" may independently comprise or consist of 1, 2, 3, 4 or 5 additional nucleosides, which may be complementary or non-complementary to the target nucleic acid. The transition between region D' or D" and the gapmer, mixmer or totalmer is recognized by the transition from a phosphorothioate linked sugar modified nucleoside to a phosphodiester linked non-sugar modified nucleoside, such as a DNA or RNA or base modified versions of these. In some embodiments the additional 5' and/or 3' end nucleotides are linked with phosphodiester linkages, both between the region D' or D" nucleosides (DNA or RNA), but also the internucleoside linkage connecting the sugar modified nucleoside at the terminal end of the gapmer, mixmer or toalmer with the additional 5' and/or 3' end nucleotides (Region D) is preferably a phosphodiester linkage. In some embodiments the additional 5' and/or 3' end nucleotides are phosphodiester linked DNA or RNA, preferably there is at least two, such as at least three, such as at least 5 consecutive phosphodiester linkages between the 5' end or the 3' end of a gapmer, mixmer or totalmer and the conjugate moiety.

Nucleotide based biocleavable linkers suitable for use as region D' or D" are disclosed in WO2014/076195, which include by way of example a phosphodiester linked DNA dinucleotide.

For the gapmer the designs using a biocleavable nucleotide linker can be described by the following formulas D'-F-G-F', F-G-F'-D" or D'-F-G-F'-D". In this instance the F-G-F' is the gapmer portion of the oligonucleotide and region D' or D" constitute a separate part of the oligonucleotide.

In one embodiment the single stranded antisense oligonucleotide to be encapsulated into the milk extracellular vesicle of the invention comprises a region D' and/or D" in addition to the contiguous nucleotide sequence which constitutes the gapmer, mixmer or totalmer.

In some embodiments, the oligonucleotide of the present invention can be represented by the following formulae:
F-G-F'; in particular F₁₋₈-G₅₋₁₆-F'₂₋₈
D'-F-G-F', in particular D'₁₋₃-F₁₋₈-G₅₋₁₆-F'₂₋₈
F-G-F'-D", in particular F₁₋₈-G₅₋₁₆-F'₂₋₈-D"₁₋₃
D'-F-G-F'-D", in particular D'₁₋₃-F₁₋₈-G₅₋₁₆-F'₂₋₈-D"₁₋₃

In some embodiments the internucleoside linkage positioned between region D' and region F is a phosphodiester linkage. In some embodiments the internucleoside linkage positioned between region F' and region D" is a phosphodiester linkage.

### Complementarity

The term "complementarity" describes the capacity for Watson-Crick base-pairing of nucleosides/nucleotides. Watson-Crick base pairs are guanine (G)-cytosine (C) and adenine (A) - thymine (T)/uracil (U). It will be understood that oligonucleotides may comprise nucleosides with modified nucleobases, for example 5-methyl cytosine is often used in place of cytosine, and as such the term complementarity encompasses Watson Crick base-paring between non-modified and modified nucleobases (see for example Hirao et al (2012) Accounts of Chemical Research vol 45 page 2055 and Bergstrom (2009) Current Protocols in Nucleic Acid Chemistry Suppl. 37 1.4.1).

The term "% complementary" as used herein, refers to the number of nucleotides in percent of a contiguous nucleotide sequence in a nucleic acid molecule (e.g. oligonucleotide) which, at a given position, are complementary to (*i.e.* form Watson Crick base pairs with) a contiguous sequence of nucleotides, at a given position of a separate nucleic acid molecule (e.g. the target nucleic acid or target sequence). The percentage is calculated by counting the number of aligned bases that form pairs between the two sequences (when aligned with the target sequence 5'-3' and the oligonucleotide sequence from 3'-5'), dividing by the total number of nucleotides in the oligonucleotide and multiplying by 100. In such a comparison a nucleobase/nucleotide which does not align (form a base pair) is termed a mismatch. Preferably, insertions and deletions are not allowed in the calculation of % complementarity of a contiguous nucleotide sequence.

The term "fully complementary", refers to 100% complementarity.

### Identity

The term "Identity" as used herein, refers to the proportion of nucleotides (expressed in percent) of a contiguous nucleotide sequence in a nucleic acid molecule (e.g. oligonucleotide) which across the contiguous nucleotide sequence, are identical to a reference sequence (e.g. a sequence motif). The percentage of identity is thus calculated by counting the number of aligned bases that are identical (a match) between two sequences (e.g. in the contiguous nucleotide sequence of the compound of the invention and in the reference sequence), dividing that number by the total number of nucleotides in the aligned region and multiplying by 100. Therefore, Percentage of Identity = (Matches x 100)/Length of aligned region (e.g. the contiguous nucleotide sequence). Insertions and deletions are not allowed in the calculation the percentage of identity of a contiguous nucleotide sequence. It will be understood that in determining identity, chemical modifications of the nucleobases are disregarded as long as the functional capacity of the nucleobase to form Watson Crick base pairing is retained (e.g. 5-methyl cytosine is considered identical to a cytosine for the purpose of calculating % identity).

### Target nucleic acid

The term "target nucleic acid" as used herein refers to a gene, a RNA, a microRNA, a mRNA, and pre-mRNA, a mature mRNA or a cDNA sequence which is intentionally modulated by a modified single-stranded antisense oligonucleotide as referred to herein. The target nucleic acid of the present invention is at least expressed in the central nervous system (e.g. brain tissue), in the spleen, in the liver and/or in T-cells. It is to be understood that the target nucleic acid is associated with the disease to be treated. Accordingly, the disease to be treated by the isolated milk extracellular vesicle as referred to herein is predicted to be improved by changing the levels of the target nucleic acid or targets directly regulated by the target nucleic acid or the protein encoded by the target nucleic acid. In one embodiment the disease is caused by abnormal levels of the target nucleic acid or a protein encoded by the target nucleic acid. In another embodiment the disease is caused by malfunctioning variants of the protein encoded by the target nucleic acid such as splice variants or mutational variants. In another embodiment the disease may be improved by increasing expression of/from the target nucleic acid compared to normal levels if such an increase for example serve to reduce abnormal levels of another disease causing protein. In particular, the disease can be improved by modulating the target nucleic acid, in the central nervous system (e.g. brain tissue), in the spleen, in the liver and/or in T-cells. The expression "abnormal level" is well understood by the skilled person and generally refers to a level of the target nucleic acid or a protein encoded from it which is increased or decreased in a subject suffering from the disease to be treated as compared to a subject not suffering from the disease (in particular, in the central nervous system (e.g. brain tissue), in the spleen, in the liver and/or in T-cells). In some embodiments the disease is treated by down-regulating expression of the target nucleic acid.

The target nucleic acid can be any nucleic acid which is expressed in the cells or tissues referred to above. In an embodiment, the target nucleic acid is a mRNA. In another embodiment, the target nucleic acid is a pre-mRNA. In another embodiment, the target nucleic acid is a long non-coding RNA (IncRNA). In another embodiment, the target nucleic acid is a miRNA.

The contiguous sequence of nucleobases of the modified single-stranded antisense oligonucleotide is typically complementary, such as 80%, such as 90%, such as 95%, such as fully complementary, to the target nucleic acid, as measured across the length of the oligonucleotide. It is advantageous it the modified single-stranded antisense oligonucleotide is fully complementary to the target nucleic acid. Optionally of one or two mismatches to the target nucleic acid can be allowed. The sequence complementarity is generally measured across the full length of the oligonucleotide or preferably accross the contiguous nucleotide sequence of the oligonucleotide optionally excluding nucleotide based linker regions which may link the oligonucleotide to an optional functional group such as a conjugate, or other non-complementary terminal nucleotides.

### Target Sequence

The term "target sequence" as used herein refers to a sequence of nucleotides present in the target nucleic acid which comprises the nucleobase sequence which is complementary to the oligonucleotide loaded into the extracellular vesicle. In some embodiments, the target sequence consists of a region on the target nucleic acid which is complementary to the contiguous nucleotide sequence of the oligonucleotide of the invention.

### Modulation of a target

The term "modulation" as used herein is to be understood as an overall term for an oligonucleotide's ability to alter the amount of target nucleic acid when compared to the amount of target nucleic acid prior to administration of the isolated milk extracellular vesicle of the present invention. Alternatively modulation of expression may be determined by reference to a control experiment. E.g., the control is an individual or target cell treated with a saline composition or unloaded isolated milk extracellular vesicle.

In one embodiment the modulation is achieved by hybridization of the modified single-stranded antisense oligonucleotide to the target nucleic acid, thereby either i) down-regulating, i.e. reducing, the expression of said target nucleic acid, ii) effecting splice switching of the target nucleic acid, resulting in expression of a splice variant of the target nucleic acid or iii) blocking the target nucleic acid, e.g. a miRNA or mRNA, to increase the protein expression from a downstream mRNA or the target mRNA.

In another embodiment the modified single-stranded antisense oligonucleotide is an aptamer which binds to a target nucleic acid or target protein via non-covalent interactions such as electrostatic interactions, hydrophobic interactions and their complementary shapes, instead of via Watson-Crick base pairing, and thereby blocks or activates it target.

The modulation of the target is at least 20% compared to the normal expression level of the target, more preferably at least 30%, at least 40%, at least 50%, at least 60%, at least 70%, at least 80%, or at least 90% compared to the normal expression level of the target.

In some embodiments the milk extracellular vesicle loaded oligonucleotide of the invention may be capable of inhibiting expression levels of the target mRNA by at least 40% *in vivo* following oral administration of extracellular vesicles with 1 µM oligonucleotide, such as 5 µM oligonucleotide.

In some embodiments the milk extracellular vesicle loaded oligonucleotide may be capable of increasing expression levels of target protein by at least 20% *in vivo* following oral administration of extracellular vesicles with 1 µM oligonucleotide, such as 5 µM oligonucleotide.

In some embodiments the milk extracellular vesicle loaded oligonucleotide of the invention may be capable of changing the splice switching of a target nucleic acid to provide an alternatively spliced target protein which constitute at least 20% of the total target protein *in vivo* following oral administration of extracellular vesicles with 1 µM oligonucleotide, such as 5 µM oligonucleotide.

### Treatment

The term 'treatment' as used herein refers to both treatment of an existing disease (*e.g*. a disease or disorder as herein referred to), or prevention of a disease, *i.e.* prophylaxis. It will therefore be recognized that treatment as referred to herein may, in some embodiments, be prophylactic. The subject to be treated is preferably a mammal, e.g. a mouse. In an embodiment, the subject is a human subject. Diseases to be treated include central nervous system diseases (such as brain diseases), spleen diseases, liver diseases and diseases involving the T-cells. Preferred diseases are disclosed elsewhere herein.

### DETAILED DESCRIPTION OF THE INVENTION

The invention provides an isolated milk extracellular vesicle carrying a modified single-stranded antisense oligonucleotide, such as a backbone-modified single-stranded antisense oligonucleotide, for use as a medicament, wherein said isolated milk extracellular vesicle is administered orally.

It has been shown in the studies in the present invention that - after oral administration of the isolated milk extracellular vesicle - the modified single-stranded antisense oligonucleotide was delivered to the central nervous system, spleen, liver and T-cells.

Accordingly, the invention provides an isolated milk extracellular vesicle carrying a modified single-stranded antisense oligonucleotide for use as a medicament, wherein said isolated milk extracellular vesicle is administered orally, wherein said modified single-stranded antisense oligonucleotide is delivered to one or more of the target tissues selected from the group consisting of the central nervous system, spleen, liver and T-cells.

Delivery of the modified single-stranded antisense oligonucleotide to the central nervous system, spleen, liver and T-cells allows for an efficient modulation of the target nucleic acid in these cells/tissues following oral administration. In particular the modulation of the target nucleic acid in the CNS, spleen and T-cells following oral administration was surprising.

Therefore, the invention provides an isolated milk extracellular vesicle carrying a modified single-stranded antisense oligonucleotide for use as a medicament, wherein said isolated milk extracellular vesicle is administered orally, wherein expression of a target nucleic acid in one or more of the target tissues selected from the group consisting of the central nervous system, spleen, liver and T-cells is modulated.

In one embodiment the modified single-stranded antisense oligonucleotide comprised by the isolated milk extracellular vesicle is single-stranded antisense oligonucleotide with at least one backbone modification selected from a modified internucleoside linkage and/or a modified sugar nucleoside.

In one embodiment the modified single-stranded antisense oligonucleotide comprised by the isolated milk extracellular vesicle is single-stranded antisense oligonucleotide with at least one modified internucleoside linkage. In an embodiment, at least 50%, such as at least 75% of the internucleoside linkages in the antisense oligonucleotide are modified internucleoside linkages. Further, it is envisaged that all internucleoside linkages of said single-stranded antisense oligonucleotide are modified internucleoside linkages.

In one embodiment the modified internucleoside linkage are selected from the group comprising phosphorothioate, diphosphorothioate and boranophosphate linkages. In particular, the modified internucleoside linkages are phosphorothioate linkages. In one embodiment all the internucleoside linkages are phosphorothioate linkages.

In one embodiment the modified single-stranded antisense oligonucleotide comprised by the isolated milk extracellular vesicle is a single-stranded antisense oligonucleotide with at least one sugar modification in the backbone. The sugar modification can for example be selected from morpholino, PNA or 2' sugar modified nucleosides.

In one embodiment, the modified single-stranded antisense oligonucleotide is a single-stranded morpholino antisense oligonucleotide.

In another embodiment, the modified single-stranded antisense oligonucleotide is single-stranded peptide nucleic acids (PNA).

In a preferred embodiment, the modified single-stranded antisense oligonucleotide comprises or consists of at least one modified internucleoside linkage and one or more 2' sugar modified nucleosides. It is advantageous if the single-stranded antisense oligonucleotide comprises at least three 2' sugar modified nucleosides, in particular located in the 5' and 3' terminal ends of the oligonucleotide or contiguous nucleotide sequence thereof. The one or more 2' sugar modified nucleosides can independently be selected from the group consisting of 2'-O-alkyl-RNA, 2'-O-methyl-RNA, 2'-alkoxy-RNA, 2'-O-methoxyethyl-RNA, 2'-amino-DNA, 2'-fluoro-DNA, arabino nucleic acid (ANA), 2'-fluoro-ANA and LNA (locked nucleic acid) nucleosides. In a preferred embodiment, the modified single-stranded antisense oligonucleotide, such as the single-stranded antisense oligonucleotides with at least one modified internucleoside linkage comprises one or more LNA (locked nucleic acid) nucleosides. Accordingly, the modified single-stranded antisense oligonucleotide is preferably a single-stranded antisense LNA oligonucleotide with at least one modified internucleoside linkage.

The modified single-stranded antisense oligonucleotide with at least one 2' sugar modified nucleoside and preferably at least one modified internucleoside linkage, is selected from the group of gapmers, mixmers, totalmers, antimiRs, blockmiRs and splice-switching oligonucleotides (SSOs). Thus, the modified single-stranded antisense oligonucleotide may be a gapmer as defined herein (such as a LNA gapmer). Alternatively, the modified single-stranded antisense oligonucleotide may be a mixmer as defined herein. Alternatively, the modified single-stranded antisense oligonucleotide may be totalmer as defined herein. Alternatively, the modified single-stranded antisense oligonucleotide may be an antimiR as defined herein. Alternatively, the modified single-stranded antisense oligonucleotide may be a splice-switching oligonucleotide as defined herein.

In particular, it is envisaged that the modified single-stranded antisense oligonucleotide is an LNA antisense oligonucleotide.

The modified single-stranded antisense oligonucleotide, e.g. the backbone modified single-stranded antisense oligonucleotide, to be loaded into the milk extracellular vesicle typically have a length of 7 to 35 nucleotides, such as 7 to 30 nucleotides. They may be in the form of a pharmaceutically acceptable salt.

In an embodiment, the modified single-stranded antisense oligonucleotide comprises a contiguous nucleotide sequence of 7 to 35 nucleotides, such as 7 to 30 nucleotides, wherein the contiguous nucleotide sequence is at least 90% complementary, such as fully complementary, to a target nucleic acid in the target tissue.

In an embodiment, the modified single-stranded antisense oligonucleotide comprises a contiguous nucleotide sequence of at least 7 to 26 nucleotides, wherein the contiguous nucleotide sequence is at least 90% complementary, such as fully complementary to a target nucleic acid in the target tissue.

In an embodiment, the modified single-stranded antisense oligonucleotide comprises a contiguous nucleotide sequence of 10 to 30 nucleotides, wherein the contiguous nucleotide sequence is at least 90% complementary, such as fully complementary to a target nucleic acid in the target tissue.

In an embodiment, the modified single-stranded antisense oligonucleotide comprises a contiguous nucleotide sequence of 7 to 14 nucleotides, wherein the contiguous nucleotide sequence is at least 90% complementary, such as fully complementary to a target nucleic acid in the target tissue.

In an embodiment, the modified single-stranded antisense oligonucleotide comprises a contiguous nucleotide sequence of 14 to 20 nucleotides, wherein the contiguous nucleotide sequence is at least 90% complementary, such as fully complementary to a target nucleic acid in the target tissue.

In an embodiment wherein the single-stranded antisense oligonucleotide is conjugated to a lipophilic conjugate moiety. Preferably the single-stranded antisense oligonucleotide includes a biocleavable nucleotide linker in the 5' terminal or 3' terminal of the oligonucleotide to which the lipophilic conjugate moiety is covalently attached.

In one embodiment the single stranded antisense oligonucleotide comprises a biocleavable nucleotide region (region D' and/or D") positioned between the contiguous nucleotide sequence of the gapmer, mixmer or totalmer and a lipophilic conjugate moiety, such as a cholesterol or tocopherol conjugate moiety.

The extracellular vesicle to be applied in the accordance with the present invention is a milk extracellular vesicle. Accordingly, it is obtained, i.e. isolated, from milk. The expressions "obtained from", "isolated from" and "derived from" are used interchangeably herein.

The milk can be the milk from any mammalian species, such as from cows, goats or sheep.

In one embodiment, the extracellular vesicle has been obtained from bovine milk. Accordingly, the extracellular vesicle is bovine milk extracellular vesicle.

In another embodiment, the extracellular vesicle has been obtained from goat milk. Accordingly, the extracellular vesicle is goat milk extracellular vesicle.

In another embodiment, the extracellular vesicle has been obtained from sheep milk. Accordingly, the extracellular vesicle is sheep milk extracellular vesicle.

The milk extracellular vesicle can be obtained from raw milk, e.g. bovine raw milk. Further, it is envisaged that the extracellular vesicle has been obtained from colostrum which is the first form of milk produced by the mammary glands of mammals immediately following delivery of the newborn. Moreover, it is envisaged that the extracellular vesicle has been obtained from ultra-heat treated (UHT) milk.

How to isolate extracellular vesicles from milk is well known in the art. For example, methods for the isolation of extracellular vesicles from milk are e.g. described in WO 2014/134132 and WO 2018/102397 which are hereby incorporated by reference with respect to the entire disclosure content. In an embodiment, milk extracellular vesicles have been obtained, i.e. isolated from milk as described in these documents. In another embodiment, milk extracellular vesicles have been obtained, i.e. isolated from milk as described the Examples section.

In particular, it is envisaged that milk extracellular vesicles have been obtained, i.e. isolated from milk, by subjecting milk to a series of sequential ultracentrifugations, optionally followed by density gradient purification. For example, the series of sequential ultracentrifugations comprises a first centrifugation for 30 min at 13'000 rcf (relative centrifugal force) and 4°C, a second ultracentrifugation at 100'000 rcf for 60 min and 4°C., and a third ultracentrifugation at 135'000 rcf for 90 min and 4°C. The first centrifugation shall allow for the remove fat globules, cells and cell debris. After centrifugation, the upper fat layer and pellet in the bottom of the tube were discarded and the supernatant is collected and subjected to the second ultracentrifugation. The supernatant after the second step can collected and filtered, e.g. sequentially through a 0.22 µm and a 0.1 µm stericup filter. The filtrate is subjected to the third ultracentrifugation step to collect milk extracellular vesicles. The supernatant can be discarded and the pellet containing milk extracellular vesicles can be resuspended in DPBS.

The milk extracellular vesicles isolated by the method described in the previous paragraph can be loaded with the modified single-stranded oligonucleotides (and could be used in therapy). Alternatively, the milk extracellular vesicles isolated by this method may be subjected to further purification steps. In particular, the milk extracellular vesicles can be subjected to density gradient purification. For example, a discontinuous sucrose/iodoxanol density gradient can be used. The medium for this density gradient is commercially available (e.g. OptiPrep Density Gradient Medium, No. D1556, Sigma Aldrich, St. Louis, MI, USA). A suitable density gradient purification has been e.g. described by Greening et al 2015, Methods Mol Biol 1295 which herewith is incorporated by reference in its entirety. In an embodiment, solutions containing 40% (w/v), 20%, 10%, and 5% iodoxanol in 0.25 M sucrose 10 mM Tris pH 7.5 are prepared and layered in ultracentrifugation tubes (e.g. 12 mL thinwall pollyalomer ultracentrifugation tubes) to obtain a discontinuous density gradient. Milk extracellular vesicles isolated by the method described in the previous paragraph are layered on top and are subsequently centrifuged for 18 hours at 100'000 rcf and 4°C. Fractions containing milk extracellular vesicles are collected (and e.g. diluted in DPBS). The obtained milk extracellular vesicles can be collected by centrifugation at 135'000 rcf for 90 min and 4°C and can be loaded with modified single-stranded oligonucleotides (see next section).

In the studies underlying the present invention, the isolation of milk extracellular vesicles included a density gradient purification step (see Examples). It was shown that the purification resulted in higher purity of bovine milk exosomes. For example, the density gradient purification step removed several soluble proteins that were present in preparations that were obtained by sequential ultracentrifugations without an additional density gradient purification step.

The milk extracellular vesicle as referred to herein shall carry, i.e. comprise, the modified single-stranded oligonucleotides as referred herein. Accordingly, the modified single-stranded antisense oligonucleotides are encapsulated in the milk extracellular vesicle. In other words, the isolated milk extracellular vesicle is loaded with the modified single-stranded oligonucleotides.

Various methods for the encapsulation/loading of small molecules into extracellular vesicles from various sources have been described in the art. For example, an overview on encapsulation/loading methods is provided in WO2017/173034 which herewith is incorporated by reference with respect to its entire disclosure content. The present invention is not limited to a specific encapsulation/loading method. Methods for encapsulating the modified single-stranded antisense oligonucleotides into the milk extracellular vesicles can be selected from electroporation, microfluidics, sonication, saponification, extrusion, or freeze/thaw cycles.

In the studies underlying the present invention, two approaches were applied for encapsulating modified single-stranded antisense oligonucleotides in milk extracellular vesicles: a) drug loading by extrusion, and b) drug loading by microfluidics. Both approaches are based on subjecting milk extracellular vesicles to shear stress. It was shown that both methods could be applied for efficiently encapsulating modified single-stranded antisense oligonucleotides in milk extracellular vesicles. However, the extrusion method resulted in higher amounts of single-stranded antisense oligonucleotides that were encapsulated in the milk extracellular vesicles as compared to the microfluidics method. Therefore, it is in particular envisaged to encapsulate the modified single-stranded antisense oligonucleotides in milk extracellular vesicles by extrusion (e.g. as described in the Examples section).

Preferably, isolated milk extracellular vesicles carrying modified single-stranded antisense oligonucleotides have a diameter, a Zeta potential and/or a polydispersity index (PDI) as described in the in the "Definition"-section under "Extracellular vesicles/milk extracellular vesicles".

The isolated milk extracellular vesicle comprises an amount of a modified single-stranded antisense oligonucleotide which allows for the modification of the target nucleic acid (in particular in the target tissue/target cell). The isolated milk extracellular vesicle carrying a modified single-stranded antisense oligonucleotide may also be said to comprise a payload of modified single-stranded antisense oligonucleotide.

Further, it is envisaged that the amount of a modified single-stranded antisense oligonucleotide present in the isolated milk extracellular vesicle is in the range of from 0.1 to 20 % by weight, preferably in the range of from 0.5 to 15 % by weight, more preferably in the range of from 1 to 15 % by weight, even more preferably in the range of from 2 to 10 % by weight, most preferably in the range of from 3 to 8 % by weight, based on the total protein concentration of the isolated milk extracellular vesicle carrying the modified single-stranded antisense oligonucleotide. The protein concentration can be determined by colorimetric assays (BCA).

It is envisioned that the milk extracellular vesicles can be used to deliver more than one single-stranded antisense oligonucleotide, such as for example one single-stranded antisense oligonucleotide hybridizing to one target (target 1) and another single-stranded antisense oligonucleotide hybridizing to a different target (target 2).

In an embodiment, the isolated milk extracellular vesicle comprises one or more (several) modified single-stranded antisense oligonucleotides which modulate expression of different target nucleic acids, such as two, three, four, or five or more different nucleic acids. Thus, the isolated milk extracellular vesicle may comprise a mixture of different modified single-stranded antisense oligonucleotides, e.g. 2 to 10 different modified single-stranded antisense oligonucleotides.

The isolated milk extracellular vesicle carrying a modified single-stranded antisense oligonucleotide as referred to herein is administered orally. Preferably, a pharmaceutical effective amount of the isolated milk extracellular vesicle carrying modified single-stranded antisense oligonucleotides is administered. Oral administration allows for delivering the isolated milk extracellular vesicle, and thus the payload of modified single-stranded antisense oligonucleotide into target tissues selected from the group consisting of the central nervous system, spleen, liver and T-cells.

The disease to be treated in accordance with the present invention is associated with expression of the target nucleic acid or can be treated by changing the expression of the target nucleic acid. Accordingly, the disease can be caused by abnormal levels of the target nucleic acid. In a preferred embodiment, the disease is associated with expression of the target nucleic acid in the central nervous system, in the spleen, in the liver and/or in T-cells. Accordingly, the disease can be caused by abnormal levels of the target nucleic acid in the central nervous system, in the spleen, in the liver and/or in T-cells. In a preferred embodiment, the disease is a disease that can be treated by reduction of the level of the target nucleic acid, in particular in the central nervous system, in the spleen, in the liver and/or in T-cells.

In an embodiment, the modified single-stranded antisense oligonucleotide is delivered to the central nervous system. The term "central nervous system" preferably encompasses the brain and the spinal cord. Specific tissues in the brain, to which the single stranded antisense oligonucleotide may be delivered encompass the cerebellum, cerebral cortex, mid brain, thalamus, hypothalamus, hippocampus, striatum, frontal temporal lobes, motor cortex and the brain stem. Specific tissues in the spinal cord are basal root ganglion, dorsal horn and ventral horns. Accordingly, the modified single-stranded antisense oligonucleotide is delivered to the brain, the spinal cord, or both to the brain and spinal cord or specific tissues thereof.

Preferably, the delivery to the central nervous system (such as to the brain) allows for the treatment of a disease selected from the group consisting of brain cancer (such as a brain tumor), a seizure disorder, a neurodegenerative disorder, a neuropsychiatric disorder and a movement disorders. More preferably, the delivery to the central nervous system (such as to the brain) allows for the treatment of a disease selected from Angelman disorder, Alexander Disease, Alzheimer's disease, Amyotrophic lateral sclerosis, Friedreich's ataxia, Huntington's disease, Lewy body disease, Parkinson's disease, Spinal muscular atrophy, Schizophrenia, Depression, Bipolar disease, Autism, Epilepsy, Frontotemporal dementia, Progressive Bulbar Palsy, Rett syndrome, Tourette syndrome, Neurofibromatosis, progressive muscular atrophy, hereditary spastic paraplegia, Pelizaeus-Merzbacher disease, Gaucher's disease, Spinocerebellar ataxia, Pagon Bird Detter syndrome; Friedreich's ataxia; Spinocerebellar ataxia, Digeorge syndrome, Dup15q syndrome, Doose Syndrome, Glut1 Deficiency Syndrome, CDKL5 Disorder, Frontal Lobe Epilepsy, Childhood Absence Epilepsy, Early Myoclonic Encephalopathy (EME), Lennox-Gastaut Syndrome (LGS), Ohtahara Syndrome, and Landau-Kleffner Syndrome. Accordingly, the aforementioned diseases can be treated by oral administration of the isolated milk exosome as referred to herein.

In an embodiment, the modified single-stranded antisense oligonucleotide is delivered to the spleen.

Preferably, the delivery to the spleen allows for the treatment of a disease selected from the group consisting of cancer such as spleen cancer, an inflammatory disease and an immunodisorder. Accordingly, the aforementioned diseases can be treated by oral administration of the isolated milk exosome as referred to herein.

In an embodiment, the modified single-stranded antisense oligonucleotide is delivered to the liver.

Preferably, the delivery to the liver allows for the treatment of a disease selected from the group consisting of liver cancer, cardiovascular diseases, coagulation cascade deficiencies, inflammatory diseases, metabolic disease and infections. More preferably, the delivery to the liver allows for the treatment of a disease selected from hepatocellular carcinoma, liver malignancies metastasized from primary cancers in other tissues, diabetes type 1, non-insulin dependent diabetes, insulin resistance, control of blood glucose levels, HDL/LDL cholesterol imbalance, atherosclerosis, , dyslipidemias, , familial combined hyperlipidaemia (FCHL), acquired hyperlipidaemia, statin-resistant hypercholesterolemia, cardiovascular disease, coronary artery disease (CAD), and coronary heart disease (CHD), non alcoholic steatohepatitis (NASH), non-alcoholic fatty liver disease, obesity, acute coronary syndrome (ACS), thrombosis, rare bleeding disor-ders, hepatitis B or C, cytomegalovirus infection, schistosomiasis infection and leptospirosis infection, malaria, fasciola infection, rheumatoid arthritis, liver fibrosis, cirrhosis, hepatic porphyria, acute intermittent porphyria (AIP), paroxysmal nocturnal hemoglobinuria (PNH), atypical hemolytic-uremic syndrome (aHUS), myasthenia gravis, neuromyelitis optica, alpha 1-antitrypsin deficiency, Cushing's Syndrome, and transthyretin-related hereditary amyloidosis. Accordingly, the aforementioned diseases can be treated by oral administration of the isolated milk exosome as referred to herein.

In an embodiment, the modified single-stranded antisense oligonucleotide is delivered to T-cell.

Preferably, the delivery to T-cells allows for the treatment of a disease selected from the group consisting of cancer, an inflammatory disease and an infection disease.

In a further aspect, the invention provides a pharmaceutical composition comprising the isolated milk extracellular vesicle of the present invention, which carries a single-stranded antisense oligonucleotide. Said pharmaceutical composition may further comprise a pharmaceutically acceptable diluent, carrier, salt and/or adjuvant. A pharmaceutically acceptable diluent includes phosphate-buffered saline (PBS) and pharmaceutically acceptable salts include, but are not limited to, sodium and potassium salts. In some embodiments the pharmaceutically acceptable diluent is sterile phosphate buffered saline.

The invention provides methods for treating a disease by oral administration of a therapeutically effective amount of the isolated milk extracellular vesicle comprising a modified single stranded antisense oligonucleotide as referred to above or the pharmaceutical composition of the invention to a subject suffering from the disease.

The invention provides methods for treating a disease in the central nervous system, spleen, liver and T-cells by oral administration of a therapeutically effective amount of the isolated milk extracellular vesicle comprising a modified single stranded antisense oligonucleotide as referred to above or the pharmaceutical composition of the invention to a subject suffering from the disease. Preferred diseases are mentioned above. Upon oral administration, the modified single-stranded antisense oligonucleotide is delivered to one or more of the target tissues selected from the group consisting of the central nervous system, spleen, liver and T-cells.

The invention also provides for the use of the isolated milk extracellular vesicle comprising a modified single stranded antisense oligonucleotide as described above for the manufacture of a medicament for the treatment of a disease. Preferred diseases are mentioned above. The definitions and explanations given above apply accordingly. Preferably, the isolated milk extracellular vesicle is administered orally, thereby delivering the modified single-stranded antisense oligonucleotide to one or more of the target tissues selected from the group consisting of the central nervous system, spleen, liver and T-cells.

### Method of manufacture

In a further aspect, the invention provides methods for manufacturing the isolated milk extracellular vesicle comprising modified single stranded antisense oligonucleotides as referred to herein in connection with the present invention. The definitions above apply accordingly.

Preferably, the method comprises the provision of milk, such as bovine raw milk, and the isolation of milk extracellular vesicles by subjecting the provided milk to a series of sequential ultracentrifugations (in particular as described herein above or in the Examples section). In a preferred embodiment, the isolated milk extracellular vesicles are subsequently subjected to density gradient purification (in particular as described herein above or in the Examples section). In a further step, the obtained milk extracellular vesicles are loaded with modified single stranded antisense oligonucleotides, e.g. by extrusion. Preferably, the single stranded antisense oligonucleotides target a target nucleic acid which is expressed in the central nervous system, in the liver, in the spleen and/or in T-cells.

Accordingly, the present invention relates to a method for manufacturing milk extracellular vesicles carrying a modified single stranded antisense oligonucleotide, said method comprising the steps of
(a) obtaining or providing a milk,
(b) isolating milk extracellular vesicles from the milk obtained or provided in step (a), and (c) encapsulating a modified single stranded antisense oligonucleotide in the milk extracellular vesicles isolated in step (b), thereby manufacturing milk extracellular vesicles carrying a modified single stranded antisense oligonucleotide.

In an embodiment, step (b) comprises the steps
(b1) subjecting the milk provided or obtained in step (a) to a series of sequential centrifugations such as to a series of sequential ultracentrifugations to obtain milk extracellular vesicles from the milk, and
(b2) purifying the milk extracellular vesicles obtained in step (b1) by density gradient purification.

In an embodiment, the series of sequential (ultra)centrifugations in step (b1) comprises a first, second and third centrifugation. In an embodiment, the centrifugations are ultracentrifugations.

The first centrifugation shall allow for the removal of fat globules, cells and cell debris from the milk. The supernatant obtained by said first centrifugation shall comprise milk extracellular vesicles. Preferably, the upper fat layer of the supernatant obtained by said first centrifugation is removed from the supernatant before subjecting the supernatant to the second centrifugation.

The second centrifugation is carried out to further purify the milk extracellular vesicles comprised by the supernatant obtained by said first centrifugation. By carrying out the second centrifugation, a supernatant is obtained comprising milk extracellular vesicles. The pellet is discarded. For further purification, the supernatant obtained by said first centrifugation can be filtered, e.g. sequentially through a 0.22 µm and a 0.1 µm stericup filter, to give a filtrate comprising milk extracellular vesicles. The supernatant obtained by the second centrifugation or the obtained filtrate is subjected to the third centrifugation.

The third centrifugation is an ultracentrifugation and shall allow for the collection of milk extracellular vesicles. Accordingly, the third centrifugation is carried out under conditions which allow for pelleting the milk extracellular vesicles comprised by the supernatant obtained by the second centrifugation or by the obtained filtrate. Accordingly, a pellet containing milk extracellular vesicles is obtained by said third centrifugation. In an embodiment, the obtained pellet is resuspended in a suitable buffer, such as phosphate-buffered saline (e.g. a buffer comprising potassium chloride (2.67 mM), potassium phosphate monobasic (1.47 mM), sodium chloride (137.93 mM), sodium phosphate dibasic (8.06 mM), herein also referred to as DPBS).

For example, the first, second and third centrifugation can be carried out as follows:
(i) a first ultracentrifugation at 13'000 rcf (relative centrifugal force), e.g. a centrifugation for 30 min at 13'000 rcf (relative centrifugal force) and 4°C,
(ii) a second ultracentrifugation for 60 min at 100'000 rcf and 4°C, e.g. an ultracentrifugation for 60 min at 100'000 rcf and 4°C, and
(iii) a third ultracentrifugation at 135'000 rcf, e.g. an ultracentrifugation at 135'000 rcf for 90 min and 4°C.

In step b2), the milk extracellular vesicles isolated in step b1) are subjected to density gradient purification, i.e. density gradient centrifugation. The density gradient centrifugation allows for a further purification of the milk extracellular vesicles isolated in step b1). In an embodiment, the density gradient applied in step b2) is a discontinuous iodoxanol density gradient, such as a discontinuous sucrose/iodoxanol density gradient. A suitable discontinuous density gradient can be obtained by layering sucrose solutions (such as 0.25 M sucrose 10 mM Tris pH 7.5) containing different concentrations of iodoxanol upon one another, e.g. in an ultracentrifugation tube. For example, a discontinuous sucrose/iodoxanol density gradient can be obtained by layering solutions containing 40% (w/v), 20% (w/v), 10% (w/v), and 5% (w/v) iodoxanol in 0.25 M sucrose 10 mM Tris pH 7.5 upon one another. The milk extracellular vesicles isolated in step b1), are layered on top of the obtained discontinuous sucrose/iodoxanol density gradient. Afterwards, the discontinuous sucrose/iodoxanol density gradient comprising the milk extracellular vesicles is subjected to an ultracentrifugation step, for example ultracentrifugation for 18 hours at 100'000 rcf and 4°C. Subsequently, the obtained milk extracellular vesicles can be collected by centrifugation at 135'000 rcf for 90 min and 4°C.

Step (c) of the above method comprises the encapsulation of a modified single stranded antisense oligonucleotide in the milk extracellular vesicles isolated in step (b). How to encapsulate the vesicles is described herein above. In an embodiment, the modified single-stranded antisense oligonucleotide is encapsulated in milk extracellular vesicles by extrusion. Below are exemplary embodiments of the manufacture method.

In an embodiment, the method for manufacturing milk extracellular vesicles carrying a modified single stranded antisense oligonucleotide comprises the steps of
(a) obtaining or providing a milk,
(b) isolating milk extracellular vesicles from the milk obtained or provided in step (a), comprising
   (b1) subjecting the milk provided or obtained in step (a) to a series of sequential ultracentrifugations to obtain milk extracellular vesicles from the milk, and
   (b2) purifying the milk extracellular vesicles obtained in step (b1) by density gradient purification,
      and
   (c) encapsulating a modified single stranded antisense oligonucleotide in the milk extracellular vesicles isolated in step (b), thereby manufacturing milk extracellular vesicles carrying a modified single stranded antisense oligonucleotide.

In an embodiment, the method for manufacturing milk extracellular vesicles carrying a modified single stranded antisense oligonucleotide comprises the steps of
(a) obtaining or providing a milk,
(b) isolating milk extracellular vesicles from the milk obtained or provided in step (a), comprising
   (b1) subjecting the milk provided or obtained in step (a) to a series of sequential ultracentrifugations to obtain milk extracellular vesicles from the milk, and
   (b2) purifying the milk extracellular vesicles obtained in step (b1) by discontinuous iodoxanol density gradient purification, and
   (c) encapsulating a modified single stranded antisense oligonucleotide in the milk extracellular vesicles isolated in step (b), thereby manufacturing isolated milk extracellular vesicles carrying a modified single stranded antisense oligonucleotide.

In an embodiment, the method for manufacturing milk extracellular vesicles carrying a modified single stranded antisense oligonucleotide comprises the steps of
(a) obtaining or providing bovine milk,
(b) isolating milk extracellular vesicles from the milk obtained or provided in step (a), comprising
   (b1) subjecting the bovine milk provided or obtained in step (a) to a series of sequential ultracentrifugations to obtain milk extracellular vesicles from the milk, and
   (b2) purifying the bovine milk extracellular vesicles obtained in step (b1) by discontinuous iodoxanol density gradient purification,
      and
   (c) encapsulating, by extrusion, a modified single stranded antisense oligonucleotide in the milk extracellular vesicles isolated in step (b), thereby manufacturing isolated milk extracellular vesicles carrying a modified single stranded antisense oligonucleotide.

In an embodiment, the method for manufacturing an isolated milk extracellular vesicle carrying a single-stranded antisense oligonucleotide comprising the steps of
(a) centrifuging ultra-heat treated milk or raw milk to remove fat globules, cells and cell debris
(b) discarding the upper fat layer and subjecting the supernatant to a series of sequential ultracentrifugations
(c) subjecting the resuspended pellet to density gradient ultracentrifugation with a gradient containing 40% (w/v), 20%, 10%, and 5% iodoxanol
(d) collecting fractions containing the isolated milk extracellular vesicles
(e) mixing isolated milk extracellular vesicles with the single-stranded antisense oligonucleotide in a ratio of 1:2 using an extruder with a pore size between 100 and 400 nM
(f) purify the isolated milk extracellular vesicle encapsulating the single-stranded antisense oligonucleotide using dialyses with a MW cutoff of 300kDa

The present invention also concerns an isolated milk extracellular vesicle carrying a modified single-stranded antisense oligonucleotide obtained or obtainable by the method for manufacturing milk extracellular vesicles carrying a modified single stranded antisense oligonucleotide.

In an embodiment of said method of manufacture of the present invention, the single-stranded antisense oligonucleotide corresponds to a single-stranded antisense oligonucleotide component described in herein above.

In an embodiment of said process or the method of manufacture of the present invention, the hydrodynamic diameter of the isolated milk extracellular vesicle is in the range of 30 to 500 nm.

In an embodiment of said process or the method of manufacture of the present invention, the isolated milk extracellular vesicle has a Zeta potential in the range of 0 to -50 mV.

In an embodiment of said process or the method of manufacture of the present invention, the isolated milk extracellular vesicle has a polydispersity index (PDI) in the range of 0 to 0.4.

The present invention also concerns an isolated milk extracellular vesicle carrying a single-stranded antisense oligonucleotide obtained by the process of the present invention, i.e. by the process for obtaining an isolated milk extracellular vesicle comprising a single-stranded antisense oligonucleotide.

### EMBODIMENTS

In the following, embodiments of the present invention are disclosed. The definitions given herein above, apply *mutatis mutandis* to these embodiments.
1. An isolated milk extracellular vesicle carrying a single-stranded antisense oligonucleotide of 7 to 35 nucleotides with at least one backbone modification for use as a medicament, wherein said isolated milk extracellular vesicle is for oral administration, and wherein said single-stranded antisense oligonucleotide is for delivery to one or more of the target tissues selected from the group consisting of the central nervous system, spleen, liver and T-cells.
2. The isolated milk extracellular vesicle for the use of embodiment 1, wherein the single-stranded antisense oligonucleotide is capable of modulating a target nucleic acid in the target tissue.
3. The isolated milk extracellular vesicle for the use of embodiments 1 or 2, wherein the single-stranded antisense oligonucleotide comprises a contiguous nucleotide sequence of at least 7 to 26 nucleotides, wherein the contiguous nucleotide sequence is at least 90% complementary to a target nucleic acid in the target tissue.
4. The isolated milk extracellular vesicle for the use of embodiment 3, wherein said contiguous nucleotide sequence is fully complementary to said target nucleic acid.
5. The isolated milk extracellular vesicle for the use of use of any one of embodiments 1 to 4, wherein the single-stranded antisense oligonucleotide comprises a contiguous nucleotide sequence of 14 to 20 nucleotides.
6. The isolated milk extracellular vesicle for the use of use of any one of embodiments 1 to 4, wherein the single-stranded antisense oligonucleotide comprises a contiguous nucleotide sequence of 7 to 14 nucleotides.
7. The isolated milk extracellular vesicle for use of any one of embodiments 1 to 6, wherein the backbone modification comprises at least one modified internucleoside linkage and/or a modified nucleoside.
8. The isolated milk extracellular vesicle for use of any one of embodiments 1 to 6, wherein the backbone modification is a modified internucleoside linkage selected from the group comprising phosphorothioate, diphosphorothioate and boranophosphate linkages.
9. The isolated milk extracellular vesicle for the use of any one of embodiments 1 to 8, wherein at least 50% of the internucleoside linkages in the antisense oligonucleotide are phosphorothioate linkages.
10. The isolated milk extracellular vesicle for the use of any one of embodiments 1 to 9, wherein all internucleoside linkages of said single-stranded antisense oligonucleotide are phosphorothioate internucleoside linkages.
11. The isolated milk extracellular vesicle for the use of any one of embodiments 1 to 10, wherein the single-stranded antisense oligonucleotide comprises one or more nucleosides having a modified sugar moiety.
12. The isolated milk extracellular vesicle for the use of embodiment 11, wherein the nucleosides having a modified sugar moiety are 2' sugar modified nucleosides.
13. The isolated milk extracellular vesicle for the use of embodiment 12, wherein the one or more 2' sugar modified nucleosides are independently selected from the group consisting of 2'-O-alkyl-RNA, 2'-O-methyl-RNA, 2'-alkoxy-RNA, 2'-O-methoxyethyl-RNA, 2'-amino-DNA, 2'-fluoro-DNA, arabino nucleic acid (ANA), 2'-fluoro-ANA and LNA (locked nucleic acid) nucleosides.
14. The isolated milk extracellular vesicle for the use of embodiment 13, wherein the 2' sugar modified nucleoside is a LNA nucleoside.
15. The isolated milk extracellular vesicle for the use of any one of embodiment 1 to 14, wherein the single-stranded antisense oligonucleotide is selected from the group consisting of gapmers, LNA gapmers, mixmers, totalmers, antimiRs, blockmiRs and splice-switching oligonucleotides (SSOs).
16. The isolated milk extracellular vesicle for the use of any one of embodiment 1 to 7, wherein the single-stranded antisense oligonucleotide is a single-stranded morpholino antisense oligonucleotide or a single-stranded peptide nucleic acid (PNA).
17. The isolated milk extracellular vesicle for the use of any one of embodiment 1 to 16, wherein the single-stranded antisense oligonucleotide is conjugated to a lipophilic conjugate moiety.
18. The isolated milk extracellular vesicle for the use of any embodiment 17, wherein the single-stranded antisense oligonucleotide comprise a biocleavable nucleotide linker region in the 5' terminal or 3' terminal to which the lipophilic conjugate moiety is covalently attached.
19. The isolated milk extracellular vesicle for the use of any embodiment 17 or 18, wherein the lipophilic conjugate moiety is selected from cholesterol or tocopherol.
20. The isolated milk extracellular vesicle for use of any one of embodiment 1 to 19, where the milk extracellular vesicle is a bovine milk extracellular vesicle, a goat milk extracellular vesicle, or a sheep milk extracellular vesicle.
21. The isolated milk extracellular vesicle for use of any one of embodiments 1 to 20, wherein the milk extracellular vesicle is obtainable from ultra-heat treated milk or raw milk.
22. The isolated milk extracellular vesicle for use of any one of embodiments 1 to 21, wherein the milk extracellular vesicle is obtainable by subjecting milk to a series of sequential ultracentrifugations, optionally followed by density gradient purification.
23. The isolated milk extracellular vesicle for use of any one of embodiments 1 to 22, wherein the encapsulation of the single-stranded antisense oligonucleotide into the milk extracellular vesicle is achievable by extrusion.
24. The isolated milk extracellular vesicle for use of any one of embodiments 1 to 23, wherein the milk extracellular vesicles carrying the single-stranded antisense oligonucleotide has a hydrodynamic diameter in the range of 30 to 500 nm.
25. The isolated milk extracellular vesicle for use of any one of embodiments 1 to 24, wherein the milk extracellular vesicle carrying the single-stranded antisense oligonucleotide has a Zeta potential in the range of 0 to -50 mV.
26. The isolated milk extracellular vesicle for use of any one of embodiments 1 to 25, wherein the milk extracellular vesicle carrying the single-stranded antisense oligonucleotide has a polydispersity index (PDI) in the range of 0 to 0.4.
27. The isolated milk extracellular vesicle for the use of any one of embodiments 1 to 26, wherein said single-stranded antisense oligonucleotide is deliverable to the central nervous system such as to the brain and/or spinal cord, and wherein the medicament is for the treatment of a disease selected from the group consisting of brain cancer (such as a brain tumor), a seizure disorder, a neurodegenerative disorder, a neuropsychiatric disorder and a movement disorders.
28. The isolated milk extracellular vesicle for the use of embodiment 27, wherein said seizure disorder, neurodegenerative disorder, neuropsychiatric disorder or movement disorders is selected from the group consisting of Angelman disorder, Alexander Disease, Alzheimer's disease, Amyotrophic lateral sclerosis, Friedreich's ataxia, Huntington's disease, Lewy body disease, Parkinson's disease, Spinal muscular atrophy, Schizophrenia, Depression, Bipolar disease, Autism, Epilepsy, Frontotemporal dementia, Progressive Bulbar Palsy, Rett syndrome, Tourette syndrome, Neurofibromatosis, progressive muscular atrophy, hereditary spastic paraplegia, Pelizaeus-Merzbacher disease, Gaucher's disease, Spinocerebellar ataxia, Pagon Bird Detter syndrome; Friedreich's ataxia; Spinocerebellar ataxia, Digeorge syndrome, Dup15q syndrome, Doose Syndrome, Glut1 Deficiency Syndrome, CDKL5 Disorder, Frontal Lobe Epilepsy, Childhood Absence Epilepsy, Early Myoclonic Encephalopathy (EME), Lennox-Gastaut Syndrome (LGS), Ohtahara Syndrome, and Landau-Kleffner Syndrome.
29. The isolated milk extracellular vesicle for use of any one of embodiments 1 to 26, wherein said single-stranded antisense oligonucleotide is deliverable to the spleen, and wherein the medicament is for the treatment of a disease selected from the group consisting of cancer such as spleen cancer, an inflammatory disease and an immunodisorder.
30. The isolated milk extracellular vesicle for use of any one of embodiments 1 to 26, wherein said single-stranded antisense oligonucleotide is deliverable to T-cells, and wherein the medicament is for the treatment of a disease selected from the group consisting of cancer, an inflammatory disease and an infection disease.
31. The isolated milk extracellular vesicle for use of any one of embodiments 1 to 26, wherein said single-stranded antisense oligonucleotide is deliverable to the liver, and wherein the medicament is for the treatment of a disease selected from the group consisting of liver cancer, cardiovascular diseases, coagulation cascade deficiencies, inflammatory diseases, metabolic disease and infections.
32. The isolated milk extracellular vesicle for use of embodiment 31, wherein the liver diseases are selected from the group consisting of hepatocellular carcinoma, liver malignancies metastasized from primary cancers in other tissues, diabetes type 1, non-insulin dependent diabetes, insulin resistance, control of blood glucose levels, HDL/LDL cholesterol imbalance, atherosclerosis, dyslipidemias, familial combined hyperlipidaemia (FCHL), acquired hyperlipidaemia, statin-resistant hypercholesterolemia, cardiovascular disease, coronary artery disease (CAD), and coronary heart disease (CHD), non alcoholic steatohepatitis (NASH), non-alcoholic fatty liver disease, obesity, acute coronary syndrome (ACS), thrombosis, rare bleeding disorders, hepatitis B or C, cytomegalovirus infection, schistosomiasis infection and leptospirosis infection, malaria, fasciola infection, rheumatoid arthritis, liver fibrosis, cirrhosis, hepatic porphyria, acute intermittent porphyria (AIP), paroxysmal nocturnal hemoglobinuria (PNH), atypical hemolytic-uremic syndrome (aHUS), myasthenia gravis, neuromyelitis optica, alpha 1-antitrypsin deficiency, Cushing's Syndrome, and transthyretin-related hereditary amyloidosis.
33. A method for manufacturing an isolated milk extracellular vesicle carrying a single-stranded antisense oligonucleotide comprising the steps of
   a) obtaining or providing bovine milk,
   b) isolating milk extracellular vesicles from the milk obtained or provided in step (a), comprising
      (b1) subjecting the bovine milk provided or obtained in step (a) to a series of sequential ultracentrifugations to obtain milk extracellular vesicles from the milk, and
      (b2) purifying the bovine milk extracellular vesicles obtained in step (b1) by discontinuous iodoxanol density gradient purification,
         and
      c) encapsulating, by extrusion, a modified single stranded antisense oligonucleotide in the milk extracellular vesicles isolated in step (b), thereby manufacturing isolated milk extracellular vesicles carrying a modified single stranded antisense oligonucleotide.
34. The method of embodiment 33, wherein the single-stranded antisense oligonucleotide corresponds to a single-stranded antisense oligonucleotide component described in embodiment 1 to 19.
35. The method of embodiment 33 or 34, wherein hydrodynamic diameter of the isolated milk extracellular vesicle is in the range of 30 to 500 nm.
36. The method of embodiment 33 to 35, wherein the isolated milk extracellular vesicle has a Zeta potential in the range of 0 to -50 mV.
37. The method of embodiment 33 to 36, wherein the isolated milk extracellular vesicle has a polydispersity index (PDI) in the range of 0 to 0.4.
38. An isolated milk extracellular vesicle carrying a single-stranded antisense oligonucleotide obtained by the process of embodiment 33 to 38.

### EXAMPLES

The invention will be merely illustrated by the following Examples. The said Examples shall, whatsoever, not be construed in a manner limiting the scope of the invention.

### Materials and Methods

### List of Antisense oligonucleotides

| SEQ ID NO | CMP ID | Compound | Target |
|---|---|---|---|
| 1 | ASO1 | GAGttacttgccaACT | Malat 1 |
| 2 | ASO1-C6 | C6ₒcₒaₒGAGttacttgccaACT | Malat 1 |

Capital letters represent beta-D-oxy LNA nucleosides, lowercase letters represent DNA nucleosides, all LNA C are 5-methyl cytosine, all internucleoside linkages are phosphorothioate internucleoside linkages unless specified, lowercase o indicate a phosphodiester internucleoside linkage

### Bovine milk extracellular vesicle isolation

Bovine milk extracellular vesicles were isolated using sequential ultracentrifugation and density gradient purification as described here.

Organic bovine raw milk was purchased from a local grocery store, was stored at 4°C and was used for extracellular vesicle (BMEV) isolation within two days. BMEVs were isolated using sequential ultracentrifugation followed by density gradient separation. All centrifugation steps were carried out in an Optima L80-XP ultracentrifuge (Beckman Coulter, Brea, CA, USA) using a fixed angle SW40 Ti rotor (sequential centrifugation) and a swing bucket TST41.14 rotor (density gradient). In a first step, raw milk was centrifuged for 30 min at 13'000 rcf and 4°C to remove fat globules, cells and cell debris. The upper fat layer and pellet in the bottom of the tube were discarded and the supernatant was collected. The supernatant was then centrifuged at 100'000 rcf for 60 min and 4°C. The supernatant was collected and filtered sequentially through a 0.22 µm and a 0.1 µm stericup filter (Merck Millipore, Burlington, MA, USA). The filtrate was finally centrifuged at 135'000 rcf for 90 min and 4°C to collect crude BMEVs. The supernatant was discarded and the pellet containing BMEVs was resuspended in DPBS (No. 14190250, Thermo Scientific, Waltham, MA, USA). BMEV pellet was washed one time by repeating the last centrifugation and resuspension step. To further purify crude BMEVs, a discontinuous sucrose/iodoxanol (OptiPrep Density Gradient Medium, No. D1556, Sigma Aldrich, St. Louis, MI, USA) density gradient was used as described recently with minor modifications (Greening et al 2015 Methods Mol Biol 1295: p. 179-209). In brief, solutions containing 40% (w/v), 20%, 10%, and 5% iodoxanol in 0.25 M sucrose 10 mM Tris pH 7.5 were prepared and layered in 12 mL thinwall pollyalomer ultracentrifugation tubes (Beckman Coulter, Brea, CA, USA) to obtain a discontinuous density gradient. Crude BMEVs in DPBS were layered on top and were subsequently centrifuged for 18 hours at 100'000 rcf and 4°C. 12 one mL fractions were collected and densities were determined using an Anton Paar density meter (Graz, Austria). Fractions containing BMEVs were diluted in DPBS and purified BMEVs were collected by centrifugation at 135'000 rcf for 90 min and 4°C. Purified BMEV pellets were resuspended in DPBS and stored at 4°C for immediate use (max. 7 days) or -80°C for maximum 3 months.

### Characterization of extracted Bovine milk extracellular vesicles

The four methods below can be used to characterize the isolated Bovine milk EV's.

### Determination of total BMEV protein content

Total protein content can be determined by bichinonic acid (BCA) assay (No. 23225, Thermo Scientific, Waltham, MA, USA) according to the manufacturers' recommendations.

### Western blot analysis of EV markers

BMEV marker proteins in crude BMEVs, density gradient fractions, and purified BMEVs were analyzed using western blot. BMEV samples in DPBS (No. 14190250, Thermo Scientific, Waltham, MA, USA) were mixed with cOmplete protease inhibitor (No. 11836145001, Roche Diagnostics, Rotkreuz, Switzerland), PhosSTOP (No. 04906837001, Roche Diagnostics, Rotkreuz, Switzerland) and RIPA buffer (No. 21-188, Merck, Kenilworth, NJ, USA) to reach a final concentration of 1 x according to manufacturers' recommendation. The samples were incubated for 30 min on ice and vortexed every 10 min. Lysed BMEV samples were then mixed with NuPAGE LDS sample buffer (No. NP0007, Thermo Scientific, Waltham, MA, USA) and NuPAGE reducing agent (No. NP0004, Thermo Scientific, Waltham, MA, USA) at a final concentration of 1x. Samples were incubated for 5 min at 94°C, spun down and placed on ice. 30 µg of total protein were loaded into precast Criterion TGX stain-free 4-15% gradient acrylamide gels (No. 5678094, Bio-Rad, Hercules, CA, USA) and proteins were separated for 20 min at 100 V and 60 min at 150 V in Tris-Glycine buffer (No. LC2675, Thermo Scientific, Waltham, MA, USA). Total proteins were imaged on a ChemiDoc imaging system (Bio-Rad, Hercules, CA, USA). Proteins were then blotted to PVDF membranes (No. IB401001, Thermo Scientific, Waltham, MA, USA) using an Iblot2 transfer system (Thermo Scientific, Waltham, MA, USA) according to the manufacturers' recommendations. Membranes were blocked in Tris-buffered saline (TBS) supplemented with 3% (w/v) skim milk (No. 70166, Sigma Aldrich, St. Louis, MI, USA) and 0.1% Tween-20 (No. P9416, Sigma Aldrich, St. Louis, MI, USA) for 1 hour at room temperature under constant agitation. Primary antibodies against TSG101 (No. ab30871, Abcam, Cambridge, UK) and CD9 (No. EPR2949, Abcam, Cambridge, UK) were prepared in TBS-T with 5% (w/v) BSA at a dilution of 1:1'000 or 1:2'500, respectively. Membranes were incubated with primary antibodies overnight at 4°C under constant agitation. Membranes were then washed three times 5 min with TBS-T and incubated with secondary HRP-conjugated antibodies against mouse IgG (No. ab6789, Abcam, Cambridge, UK) or rabbit IgG (No. ab97051, Abcam, Cambridge, UK) in 3% skim milk in TBS-T at a dilution of 1:3'000 for 1.5 hours at room temperature under constant agitation. Antibodies were discarded and membranes were washed three times 5 min with TBS-T. Proteins were detected after incubation with HRP substrate solution (Lumi-Light, No. 12015200001, Roche Diagnostics, Rotkreuz, Switzerland) using a ChemiDoc imaging system (Bio-Rad, Hercules, CA, USA).

### Analysis of hydrodynamic diameter and zeta potential bv dynamic and electrophoretic light scattering

Mean hydrodynamic diameter of BMEVs was determined by dynamic light scattering (DLS) using a Delsa Nano C Particle Analyzer (Beckman Coulter, Brea, CA, USA) at a laser wavelength of 685 nm. Scattered light was detected at an angle of 165°. Data was converted using CONTIN particle size distribution analysis. Results are expressed as mean ± SD of *n* = 3 measurements in DPBS (No. 14190250, Thermo Scientific, Waltham, MA, USA) at room temperature.

Zeta potential of BMEVs was determined using a Zetasizer ZSP (Malvern Instruments, Malvern, UK) according to the manufacturers' recommendations. Samples were measured in 0.1 DPBS at 25°C. Results are shown as mean ± SD of *n* = 3 measurements.

### Analysis of particle morphology by transmission electron microscopy

Particle morphology was analyzed using transmission electron microscopy (TEM) after uranyl acetate negative staining. Formvar coated copper grids were glow discharged and incubated with 5 µL of BMEV samples in DPBS (No. 14190250, Thermo Scientific, Waltham, MA, USA) for 1 min. Samples were removed and grids were washed two times with ultra-pure water (No. 10977023, Thermo Scientific, Waltham, MA, USA). Grids were then washed once using 2% uranyl acetate and were subsequently incubated in 2% uranyl acetate for 15 sec. Uranyl acetate was removed and grids were allowed to dry before analysis. Images were acquired using a JEM 1400 transmission electron microscope (JEOL, Tokyo, Japan).

### Loading Locked nucleic acid antisense oligonucleotide (LNA ASO) into Bovine milk EV's

LNA ASOs were encapsulated in BMEVs by shear stress. Drug loaded BMEVs were used within one week after preparation and purification. One of the following two approaches was applied.

### Drug loading bv extrusion

BMEVs were mixed with LNA in DPBS (No. 14190250, Thermo Scientific, Waltham, MA, USA) and extruded at 50°C using an Avanti Polar Lipids hand extruder (Alabaster, AL, USA). Different pore sizes were tested (100 nm, 200 nm, 400 nm) and drug loading was performed at different LNA ASO-to-BMEV ratios (LNA ASO per total BMEV protein) ranging from 1 to 4. Maximum loading was achieved at a total BMEV protein concentration of 2 mg/mL, 200 nm pores, and a LNA ASO-to-BMEV ratio of 2. Drug loaded BMEVs were purified by dialysis for 72 hours at 4°C against DPBS using a molecular weight cut-off (MWCO) of 300 kDa (Float-a-lyzer, No. G235036, Repligen, Waltham, MA, USA).

### Drug loading bv microfluidics

BMEVs were mixed with LNA ASOs in DPBS (No. 14190250, Thermo Scientific, Waltham, MA, USA). The BMEV LNA ASO mixture was placed in a plastic syringe (Becton Dickinson Company, Franklin Lakes ,NJ, USA) and mixed with 75% (v/v) ethanol/DPBS using a NanoAssemblr benchtop microfluidics device (Precision Nanosystems, Vancouver, BC, Canada). Microfluidic mixing was performed at a flow rate ratio (FRR) or 2:1 and a total flow rate of 10 mL/min. LNA ASO loaded BMEVs in 25% (v/v) ethanol/DPBS were collected and incubated for another 15 min at room temperature. Ethanol and non-encapsulated LNA ASOs were removed by dialysis against DPBS for 72 hours at 4°C using a MWCO of 300 kDa (Float-a-lyzer, No. G235036, Repligen, Waltham, MA, USA).

### Characterization of LNA ASO loaded BMEVs

BMEVs loaded with LNA ASO can be characterized by the following analysis's

### Analysis of purification

Removal of non-encapsulated LNA ASOs can be confirmed using agarose gel electrophoresis. 20 µL of LNA ASO loaded BMEVs are sonicated for three times 5 min at an amplitude of 75 to release LNA ASO cargo (encapsulated LNA ASOs). 20 µL of non-treated LNA ASO loaded BMEVs, 20 µL of sonicated LNA ASO loaded BMEVs and LNA ASO standards in DPBS are loaded into a SYBR Green labeled 2% agarose gel (No. G521802, Thermo Scientific, Waltham, MA, USA) and nucleic acids are separated for 10 min. Gels are then imaged using an E-Gel imager (Thermo Scientific. Waltham, MA, USA).

### Quantification of LNA ASO drug loading content

Drug loading content was analyzed using HPLC (Water, Milford, MA, USA). Samples were injected on a C18 column (XBridge BEH C18 2.5 µm, 4.6 x 100 mm Column XP; No. 186006039, Waters, Milford, MA, USA) and samples were run on an acetonitrile/200 mM acetate gradient at a column temperature of 50°C. LNA ASOs were detected at a wavelength of 260 nm. A calibration curve of LNA ASOs was prepared and used to quantify amount of encapsulated LNA ASOs.

### In vitro cell cultures

### human hepatocytes

Primary human cryopreserved hepatocytes (PHH, No. F00995, BioIVT, West Sussex, UK) were cultured in Williams Medium E (No. W1878, Sigma Aldrich, St. Louis, MI, USA) supplemented with 10 U/mL Penicillin and Streptomycin (No. 15140122, Thermo Scientific, Waltham, MA, USA) and 10% FBS (No. 97068-085, VWR International, Radnor, PA, USA) at 37°C and 5% CO₂. 4 x 10⁴ cells were plated on collaged coated (No. 356407, BD Biosciences, Franklin Lakes, NJ, USA) 96-well culture plates (No. 3474, Corning, Corning, NY, USA). Cells were allowed to adhere for 4 hours before addition of test substances.

### H460 lung cancer cells

NCI-H460 human lung cancer cells were cultured in RPMI-1640 medium (No. 11875093, Thermo Scientific, Waltham, MA, USA) supplemented with 10% FBS (No. A3160401, Thermo Scientific, Waltham, MA, USA) at 37°C and 5% CO₂. For knockdown experiments, cells were washed once with DPBS (No. 14190250, Thermo Scientific, Waltham, MA, USA) and detached using 0.25% Trypsin-EDTA (No. 25200056, Thermo Scientific, Waltham, MA, USA). Complete culture medium was added and cells were collected by centrifugation at 1300 rpm and 5 min. The supernatant was aspirated and the cell pellet was resuspended in complete culture medium. Dead cells were stained using 0.4% Trypan blue (No. T10282, Thermo Scientific, Waltham, MA, USA) and cells were counted using a Countess II FL Automated Cell Counter (Thermo Scientific, Waltham, MA, USA). Cells were then seeded in a 96-well plate (No. 3474, Corning, Corning, NY, USA) at a density of 2 x 10⁴ cells per well and allowed to adhere overnight before addition of test substances.

### CaCo-2 cells

CaCo-2 cells were cultured in DMEM (No. 11965092, Thermo Scientific, Waltham, MA, USA) supplemented with 10% FBS and 1 x non-essential amino acids (No. 11140076, Thermo Sicentific, Waltham, MA, USA). For knockdown experiments, cells were washed once with DPBS (No. 14190250, Thermo Scientific, Waltham, MA, USA) and detached using 0.25% Trypsin-EDTA (No. 25200056, Thermo Scientific, Waltham, MA, USA). Complete culture medium was added and cells were collected by centrifugation at 1300 rpm and 5 min. The supernatant was aspirated and the cell pellet was resuspended in complete culture medium. Dead cells were stained using 0.4% Trypan blue (No. T10282, Thermo Scientific, Waltham, MA, USA) and cells were counted using a Countess II FL Automated Cell Counter (Thermo Scientific, Waltham, MA, USA). Cells were then seeded in a 96-well plate (No. 3474, Corning, Corning, NY, USA) at a density of 2 x 10⁴ cells per well and allowed to adhere overnight before addition of test substances.

### Lactat dehydrogenase assay

PHH cell culture supernatants were collected 48 hours after incubation with BMEVs and LDH release was determined using the Cytotoxicity Detection kit (No. 11644793001, Roche Diagnostics, Rotkreuz, Switzerland) according to the manufacturers' recommendations. In brief, collected cell culture supernatants were diluted and transferred to clear bottom 96-well plates (No. 3906, Corning, Corning, NY, USA). Cytotoxicity Detection reaction mix was freshly prepared and added to each well, plates were shaked at 1000 rpm for 1 min and incubated for 30 min at room temperature. Absorbance was measured at 490 nm using an EnSpire plate reader (Perkin Elmer, Waltham, MA, USA). LDH concentration was determined using Precipath U (No. 10171778122, Roche Diagnostics, Rotkreuz, Switzerland) as LDH standards. DPBS treated cells were used as control. Results are expressed as mean ± SD of *n* = 4 technical replicates.

### Intracellular ATP content

Intracellular ATP content in BMEV treated PHHs was determined 48 hours after incubation using Cell Titer Glo kit (No. G7571, Promega, Madison, WI, USA) according to the manufacturers' instruction. In brief, 50 µL of Cell Titer Glo reagent was added to each well containing treated PHHs (see section 3.11) in 50 µL of complete culture medium and plates were shaked for 2 min to induce cell lysis and plates were incubated for 10 min at room temperature. 70 µL of cell lysates were transferred into a white opaque 96-well plate (No. 3601, Corning, Corning, NY, USA) and luminescence was collected on a EnVision plate reader (Perkin Elmer, Waltham, MA, USA). Dilutions of ATP in complete culture medium were used as standards. DPBS treated cells were used as control. Results are expressed as mean ± SD of *n* = 4 technical replicates.

### Albumin release assay

Albumin secretion by PHH after BMEV treatment was determined 48 hours after incubation using the AlphaLISA biotin-free human serum albumin detection kit (No. AL363, Perkin Elmer, Waltham, MA, USA) according to the manufacturers' recommendation. Results are shown as mean ± SD of *n* = 4 technical replicates.

### ASO concentrations in tissue homogenates using hybridization ELISA (hELISA)

The general method is described in Straarup et al 2010 Nucleic Acids Res, 38(20): p. 7100-11. In brief, capture detection solution (CDS) was prepared by supplementing 5x SSC buffer (No. 93017, Sigma Aldrich, St. Louis, MI, USA) with 0.05% Tween-20 (No. P9416, Sigma Aldrich, St. Louis, MI, USA) and adding biotinylated capture probe and digoxigenin conjugated detection probe at a final concentration of 35 nM (RTR No. 35148-3 and No. 31443-7, Roche Innovation Center Copenhagen, Hørsholm, Denmark). Streptavidin coated 96-well plates (No. 436014, Thermo Scientific, Waltham, MA, USA) were washed three times with 5x SSC-T buffer and 100 µL of tissue homogenates (diluted 1:10 in CDS) or RTR17293 standards in CDS were added. Samples were incubated for 1 hour at room temperature under constant agitation. Plates were washed three times with 2x SSCT buffer. Wells were incubated with alkaline phosphatase conjugated anti-digoxigenin antibody (No. 11093274910, Roche Diagnostics, Rotkreuz, Switzerland) diluted 1:3'000 in DPBS supplemented with 0.05% Tween-20 for 1 hour at room temperature under constant agitation. Plates were washed three times with 2x SSCT buffer. Blue Phos substrate (No. 55-88-02, Seracare Life Sciences, Milford, MA, USA) was mixed according to manufacturer's instructions and 100 µL of substrate were added to each well. Absorbance was measured at a wavelength of 600 nm using GlowMax Discoverer plate reader (Promega, Madison, WI, USA) and unknown concentrations were determined by preparing a sigmoidal (4PL) standard curve of standards using GraphPad Prism Version 6 .07 (GraphPad Software, La Jolla, CA, USA). Results are expressed as mean ± SD of *n* = 4 replicates.

### Example 1 Preparation and characterization of Bovine milk extracellular vesicle (EV)

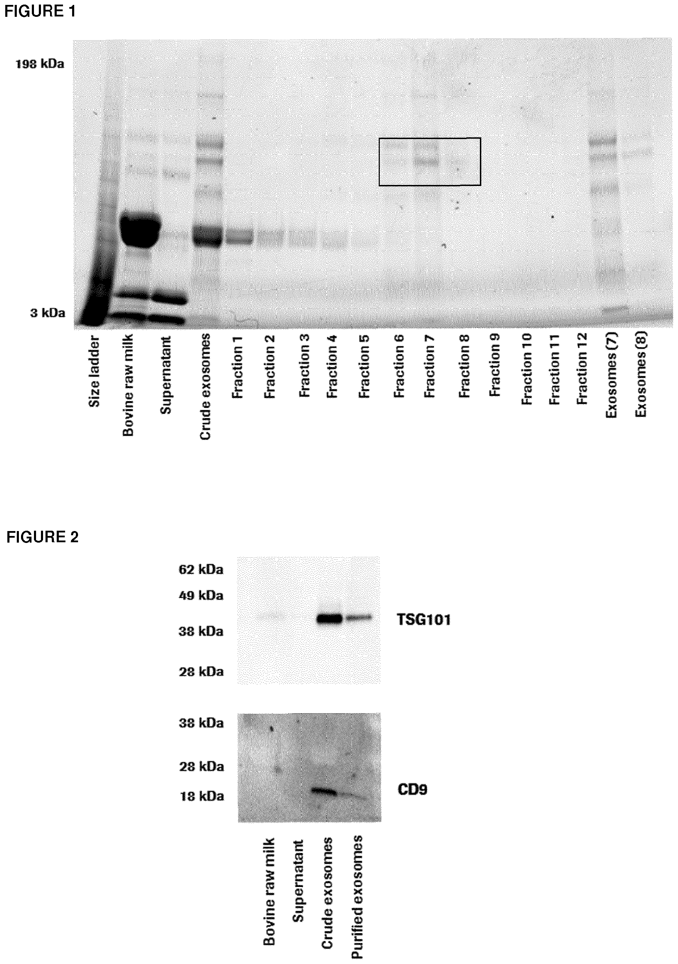

Bovine milk extracellular vesicles (BMEVs) were isolated as described in the Materials and Method section. The raw milk, crude exosome fraction and ultracentrifugation fractions from the isolation process were run on a SDS page presented in figure 1. From this it can be seen that the purified exosomes are present in fraction 6 to 8 of the ultracentrifugation (framed by the black box).

The BMEVs were characterized by some of the methods in the Material and method section. Western blot analysis (figure 2) verified the presence of the extracellular vesicle biomarkers CD9 and TSG101.

The BMEV's were also characterized in terms of their physico-chemical properties. The results are shown in table 2 in example 3 below. From these data the BMEVs are characterized as monodisperse with a slight negative surface charge.

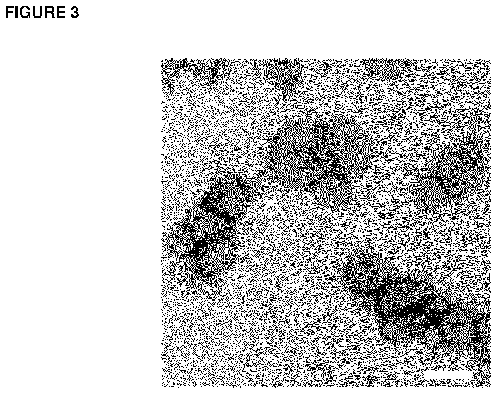

Transmission electron micrograph of extracellular vesicles are presented in figure 3.

### Example 2 In vitro evaluation of isolated BMEV's

It was investigated whether the purified BMEVs from example 1 were tolerated in an *in vitro* cell culture.

Primary human cryopreserved hepatocytes were cultivated as described in the Materials and Methods section. BMEVs were diluted in FBS free culture medium to reach final assay concentrations of 0.04 and 0.4 mg/mL total BMEV protein. BMEVs were added to wells and PHHs were incubated for 24 hours at 37°C. Medium was exchanged with complete culture medium and PHHs were incubated for another 24 hours at 37°C.

The tolerability was assessed using lactate dehydrogenase assay, intracellular ATP content and albumin release assay, all described in the Materials and Methods section.

The results are shown in table 1, and clearly indicate that the BMEVs are well tolerated by the PPH cells.

**Table 1. In vitro toxicity evaluation on cryopreserved primary human hepatocytes (PHHs). Results are shown as mean ± SD of n = 4 technical replicates.**

| | **DPBS** | **BMEVs** | |
|---|---|---|---|
| | | 0.04 mg/mL | 0.4 mg/mL |
| Lactate dehydrogenase concentration in culture medium supernatant [mU/mL] | 206 ± 18 | 193 ± 18 | 200 ± 16 |
| Intracellular ATP content [pmol/well] | 172 ± 5 | 171 ± 6 | 161 ± 7 |
| Albumin concentration in culture medium supernatant [µg/mL] | 0.47 ± 0.03 | 0.42 ± 0.04 | 0.40 ± 0.01 |

### Example 3 Loading of LNA ASO into BMEV

The BMEV's from example 1 were loaded with ASO1 or ASO1-C6 using the extrusion method described in the Materials and Methods section.

The physico-chemical properties as well as the LNA ASO content in the loaded BMEVs was analyzed as describe in the Materials and Methods section. The results of BMEVs loaded with ASO1 are shown in table 2 below.

**Table 2. Physico-chemical characterization of bovine milk extracellular vesicles. Results are shown as mean ± SD of n = 3 experiments.**

| | **BMEVs** | **ASO1 loaded BMEVs** |
|---|---|---|
| Hydrodynamic diameter (Dh) [nm] | 189.6 ± 8.3 | 163.0 ± 9.3 |
| Polydispersity index (PDI) | 0.191 ± 0.049 | 0.158 ± 0.037 |
| Zeta potential [mV]¹ | -23.1 ± 5.35 | -27.2 ± 6.77 |
| LNA ASO loading content [% w/w]² | - | 5.6 ± 0.2 |
| ¹ Determined in 0.1 DPBS (0.81 mM phosphate, 13.7 mM NaCl) | | |
| ² LNA ASO content per total BMEV protein content | | |

### Example 4 In vitro evaluation of LNA ASO-Ioaded BMEVs in H460 cells

BMEV's loaded with ASO1-C6 as described in example 3 were tested in H460 lung cancer cells to assess whether they are tolerated in an *in vitro* cell culture and whether a reduction of the target can be achieved.

The H460 lung cancer cells were cultivated as described in the Materials and Method section. The cells were treated with ASO1-C6 in DPBS (No. 14190250, Thermo Scientific, Waltham, MA, USA) or ASO1-C6 encapsulated in BMEVs with the following LNA-ASO concentration 10, 50, 100, 500, 1'000, and 3'500 nM. Cells were incubated for 24 hours at 37°C. Then, the medium was aspirated, cells were washed once with DPBS (No. 14190250, Thermo Scientific, Waltham, MA, USA), and incubated with fresh complete culture medium for another 24 hours. 48 hours after incubation, cells were washed twice with DPBS (No. 14190250, Thermo Scientific, Waltham, MA, USA).

RNA was isolated using the PureLink Pro 96 total RNA Purification kit (No. 12173011A, Thermo Scientific, Waltham, MA, USA). RNA expression was analyzed using the LightCycler Multiplex RNA Virus Master kit (No. 07083173001, Roche Diagnostics, Rotkreuz, Switzerland) on a LightCycler 480 instrument. Malat-1 IncRNA (Hs00273907_s1) and GAPDH (Hs02786624_g1) primers were ordered from Thermo Scientific (Waltham, MA, USA). Malat-1 IncRNA expression was analyzed by ΔΔct method against GAPDH. Results are expressed as mean fold-change ± SD of *n* = 4 technical replicates.

The results are shown in table 3, and indicate that malat-1 target knockdown can be achieved *in vitro* with the LNA-ASO-Ioaded BMEVs although it seems to be less efficient than for the DPBS formulated LNA-ASO.

**Table 3. In vitro evaluation of LNA-ASO loaded BMEVs in H460 lung cancer cells. Results are shown as percentage of control mean ± SD of n = 4 technical replicates.**

| **Concentration ASO1-C6 [nM]** | **ASO1-C6 in DPBS** | **ASO1-C6 in BMEVs** |
|---|---|---|
| 10 | 111 ± 10 | 92 ± 39 |
| 50 | 104 ± 27 | 109 ± 25 |
| 100 | 79 ± 8 | 118 ± 16 |
| 500 | 66 ± 6 | 76 ± 24 |
| 1000 | 59 ± 11 | 72 ± 7 |
| 3500 | 22 ± 3 | 51 ± 12 |

### Example 5 In vitro evaluation of LNA ASO-Ioaded BMEVs in CaCO-2 cells

BMEV's loaded with ASO1 as described in example 3 were tested in CaCo-2 cells to assess whether they are tolerated in an *in vitro* cell culture and whether a reduction of the target can be achieved.

The CaCo-2 cells were cultivated as described in the Materials and Methods section. The cells were treated with ASO1 in DPBS (No. 14190250, Thermo Scientific, Waltham, MA, USA) or ASO1 encapsulated in BMEVs with the following LNA-ASO concentration 10, 50, 100, 500 and 1'000 nM. Cells were incubated for 24 hours at 37°C. Then, the medium was aspirated, cells were washed once with DPBS (No. 14190250, Thermo Scientific, Waltham, MA, USA), and incubated with fresh complete culture medium for another 24 hours. 48 hours after incubation, cells were washed twice with DPBS (No. 14190250, Thermo Scientific, Waltham, MA, USA).

RNA was isolated using the PureLink Pro 96 total RNA Purification kit (No. 12173011A, Thermo Scientific, Waltham, MA, USA). RNA expression was analyzed using the LightCycler Multiplex RNA Virus Master kit (No. 07083173001, Roche Diagnostics, Rotkreuz, Switzerland) on a LightCycler 480 instrument. Malat-1 IncRNA (Hs00273907_s1) and GAPDH (Hs02786624_g1) primers were ordered from Thermo Scientific (Waltham, MA, USA). Malat-1 IncRNA expression was analyzed by ΔΔct method against GAPDH. Results are expressed as mean fold-change ± SD of *n* = 4 technical replicates.

The results are shown in table 4, and indicate that malat-1 target knockdown can be achieved *in vitro* with the LNA-ASO-Ioaded BMEVs although it seems to be less efficient than for the DPBS formulated LNA-ASO.

**Table 4. In vitro evaluation of LNA-ASO loaded BMEVs in CaCo-2 cells Results are shown as mean percentage of control ± SD of n = 4 technical replicates.**

| **Concentration ASO1 [nM]** | **ASO1 in DPBS** | **ASO1 in BMEVs** |
|---|---|---|
| 10 | 76 ± 7 | 83 ± 10 |
| 50 | 83 ± 13 | 98 ± 16 |
| 100 | 64 ± 7 | 82 ± 23 |
| 500 | 71 ± 11 | 50 ± 8 |
| 1000 | 86 ± 34 | 67 ± 30 |

### Example 6 in vivo evaluation of LNA-ASO-Ioaded BMEV's

The biodistribution and ability to achieve target reduction of the LNA-ASO-Ioaded BMEV's was asses *in vivo* in mice using either intravenous injection or oral administration.

All animal experiments were performed according to Swiss animal protection legislation and in accordance with international guidelines and good practices.

C57BL6 mice were administered with ASO1 in DPBS or ASO1 encapsulated in BMEVs as described in example 3. The administration protocol is summarized in the table below

| **Group (No of animals)** | **Treatment** | **Application route** | **Dose ASO1 [mg/kg BW]** |
|---|---|---|---|
| 1 (2) | Vehicle control (DPBS) | i.v. bolus injection | - |
| 2 (2) | Vehicle control (BMEV) | Oral gavage | - |
| 3 (4) | ASO1 in BMEVs | i.v. bolus injection | 0.485 |
| 4 (4) | ASO1 in DPBS | i.v. bolus injection | 0.425 |
| 5 (4) | ASO1 in BMEVs | Oral gavage | 0.85 |

Mice were sacrificed 3 days after treatment, organs were collected and rinsed in 0.9% NaCl. Spleens were dissected in two pieces and one half was placed in RPMI-1640 culture medium (No. 11875093, Thermo Scientific, Waltham, MA, USA) at 4°C for immediate T-cell isolation (see below). All other organs were stored in RNAlater solution (No. AM7021, Thermo Scientific, Waltham, MA, USA) at -80°C until further processing. Organs were placed in 3x volumes of QIAzol lysis reagent (No. 79306, Qiagen, Hilden, Germany), transferred into 2 mL or 5 mL Precellys tubes (No. KT03961-1-002.2 or No. KT03961-1-302.7, Bertin Instruments, Montigny-le-Bretonneux, France) containing ceramic beads, and homogenized using a Precellys evolution homogenizer (Bertin Instruments, Montigny-le-Bretonneux, France). ASO1 concentrations in tissue homogenates were determined using hybridization ELISA (hELISA) as described in the Materials and Method section. The biodistribution of ASO 1 is show in in table 5, clearly indicating distribution of the LNA_ASO-Ioaded MBVEs to several tissues both when given I.V. and by oral gavge. With I.V. administration appearing to provide the largest oligonucleotide concentration.

**Table 5. In vivo biodistribution evaluation of ASO1. Concentrations of ASO1 in the indicated tissues is in nM and shown as mean ± SD of n = 4 replicates.**

| **Treatment** | **ASO1 in DPBS** | **ASO1 in BMEVs** | **ASO1 in BMEVs** |
|---|---|---|---|
| Administration route | i.v. injection | i.v. injection | p.o. gavage |
| Liver | 6.5 ± 3.6 | 5.1 ± 1.6 | 0.4 |
| Kidney left | 14.1 ± 0.7 | 13.6 ± 4.1 | 1.5 ± 0.7 |
| Kidney right | 14.7 ± 1.2 | 13.1 ± 2.5 | 1.8 ± 1.0 |
| Small intestine | 1.8 ± 0.4 | 2.1 ± 1.7 | 6.3 |
| Brain | nd | nd | nd |
| Spleen | 3.6 ± 0.9 | 3.7 ± 2.2 | 1.3 |
| T-cells | nd | nd | nd |

| | | | |
|---|---|---|---|
| nd= not detectable by hELISA | | | |

The knockdown of target RNA (Malat-1 mRNA) was also analyzed in the collected tissues by RT-PCR. In brief total RNA was isolated from tissue homogenates using the miRNeasy Mini Kit (No. 217004, Qiagen, Hilden, Germany) according to the manufacturers' recommendations. Total RNA was quantified using the Quant-iT RiboGreen RNA Assay Kit (R11490, Thermo Scientific, Waltham, MA, USA). RNA expression was analyzed using the LightCycler Multiplex RNA Virus Master kit (No. 07083173001, Roche Diagnostics, Rotkreuz, Switzerland) on a LightCycler 480 instrument (Roche Diagnostics, Rotkreuz, Switzerland). Malat-1 IncRNA (Mm01227912_s1) and GAPDH (Mm99999915_g1) primers were ordered from Thermo Scientific (Waltham, MA, USA). Malat-1 mRNA expression was analyzed by ΔΔct method against GAPDH/normalized to total RNA content. The results are shown in table 6 and are expressed as mean fold-change ± SD of *n* = 4 replicates. The results shows that target reduction can be achieved in all tested tissues except for the small intestine. The LNA ASO-loaded BMEV's administered by oral gavage seem to result in a very efficient target knock down in the brain and the spleen. In T-cells the target knock down is better for the BMEVs given orally than for those injected by I.V.

**Table 6. In vivo target knock-down efficiency of ASO1. Expression values are shown as fold-change of control (DPBS treated animals) ± SD of n = 4 replicates.**

| **Treatment** | **DPBS** | **ASO1 in DPBS** | **ASO1 in BMEVs** | **ASO1 in BMEVs** |
|---|---|---|---|---|
| Administration route | i.v. injection | i.v. injection | i.v. injection | p.o. gavage |
| Liver | 103 ± 23 | 47 ± 8 | 56 ± 12 | 71 ± 18 |
| Kidney left | 104 ± 28 | 58 ± 10 | 57 ± 12 | 57 ± 8 |
| Kidney right | 100 ± 3 | 54 ± 5 | 56 ± 18 | 65 ± 14 |
| Small intestine | 101 ± 17 | 109 ± 32 | 114 ± 15 | 123 ± 25 |
| Brain | 101 ± 11 | 71 ± 22 | 84 ± 7 | 34 ± 10 |
| Spleen | 100 ± 1 | 37 ± 22 | 76 ± 37 | 11 ± 2 |
| T-cells | 100 ± 8 | 83 ± 37 | 92 ± 10 | 74 ± 3 |

## Claims

1. An isolated milk extracellular vesicle carrying a single-stranded antisense oligonucleotide of 7 to 35 nucleotides with at least one backbone modification for use as a medicament, wherein said isolated milk extracellular vesicle is for oral administration, and wherein said single-stranded antisense oligonucleotide is for delivery to one or more of the target tissues selected from the group consisting of the central nervous system, spleen, liver and T-cells.

2. The isolated milk extracellular vesicle for the use of claim 1, wherein the single-stranded antisense oligonucleotide is capable of modulating a target nucleic acid in the target tissue.

3. The isolated milk extracellular vesicle for the use of claims 1 or 2, wherein the single-stranded antisense oligonucleotide comprises a contiguous nucleotide sequence of at least 7 to 26 nucleotides, such as a contiguous nucleotide sequence of 7 to 14 nucleotides, or of 14 to 20 nucleotides, wherein the contiguous nucleotide sequence is at least 90% complementary, such as fully complementary to a target nucleic acid in the target tissue.

4. The isolated milk extracellular vesicle for use of any one of claims 1 to 3, wherein the backbone modification comprises at least one modified internucleoside linkage and/or a modified nucleoside.

5. The isolated milk extracellular vesicle for use of any one of claims 1 to 4, wherein the backbone modification is a modified internucleoside linkage selected from the group comprising phosphorothioate, diphosphorothioate and boranophosphate linkages.

6. The isolated milk extracellular vesicle for the use of any one of claims 1 to 5, wherein at least 50% of the internucleoside linkages in the antisense oligonucleotide are phosphorothioate linkages, and/or wherein all internucleoside linkages of said single-stranded antisense oligonucleotide are phosphorothioate internucleoside linkages.

7. The isolated milk extracellular vesicle for the use of any one of claims 1 to 6, wherein the single-stranded antisense oligonucleotide comprises one or more nucleosides having a modified sugar moiety.

8. The isolated milk extracellular vesicle for the use of claim 7, wherein the nucleosides having a modified sugar moiety are 2' sugar modified nucleosides such as 2' sugar modified nucleosides independently selected from the group consisting of 2'-O-alkyl-RNA, 2'-O-methyl-RNA, 2'-alkoxy-RNA, 2'-O-methoxyethyl-RNA, 2'-amino-DNA, 2'-fluoro-DNA, arabino nucleic acid (ANA), 2'-fluoro-ANA and LNA (locked nucleic acid) nucleosides.

9. The isolated milk extracellular vesicle for the use of any one of claim 1 to 8, wherein the single-stranded antisense oligonucleotide is selected from the group consisting of gapmers, LNA gapmers, mixmers, totalmers, antimiRs, blockmiRs and splice-switching oligonucleotides (SSOs).

10. The isolated milk extracellular vesicle for the use of any one of claim 1 to 4, wherein the single-stranded antisense oligonucleotide is a single-stranded morpholino antisense oligonucleotide or a single-stranded peptide nucleic acid (PNA).

11. The isolated milk extracellular vesicle for the use of any one of claim 1 to 10, wherein the single-stranded antisense oligonucleotide is conjugated to a lipophilic conjugate moiety by covalently attaching the lipophilic conjugate moiety to a biocleavable nucleotide linker region in the 5' terminal or 3' terminal of the single-stranded antisense oligonucleotide.

12. The isolated milk extracellular vesicle for the use of any claim 11, wherein the lipophilic conjugate moiety is selected from cholesterol or tocopherol.

13. The isolated milk extracellular vesicle for use of any one of claim 1 to 12, wherein the milk extracellular vesicle is a bovine milk extracellular vesicle, a goat milk extracellular vesicle, or a sheep milk extracellular vesicle, and/or wherein the milk extracellular vesicle is obtainable from ultra-heat treated milk or raw milk.

14. The isolated milk extracellular vesicle for use of any one of claims 1 to 13, wherein the milk extracellular vesicle is obtainable by subjecting milk to a series of sequential ultracentrifugations, optionally followed by density gradient purification.

15. The isolated milk extracellular vesicle for use of any one of claims 1 to 14, wherein the encapsulation of the single-stranded antisense oligonucleotide into the milk extracellular vesicle is achievable by extrusion.

16. The isolated milk extracellular vesicle for use of any one of claims 1 to 15, wherein the milk extracellular vesicle carrying the single-stranded antisense oligonucleotide has a hydrodynamic diameter in the range of 30 to 500 nm, a Zeta potential in the range of 0 to 50 mV, and/or a polydispersity index (PDI) in the range of 0 to 0.4.

17. The isolated milk extracellular vesicle for the use of any one of claims 1 to 16, wherein
a) said single-stranded antisense oligonucleotide is deliverable to the central nervous system such as to the brain and/or spinal cord, and wherein the medicament is for the treatment of a disease selected from the group consisting of brain cancer (such as a brain tumor), a seizure disorder, a neurodegenerative disorder, a neuropsychiatric disorder and a movement disorders, such as seizure disorder, neurodegenerative disorder, neuropsychiatric disorder or movement disorders is selected from the group consisting of Angelman disorder, Alexander Disease, Alzheimer's disease, Amyotrophic lateral sclerosis, Friedreich's ataxia, Huntington's disease, Lewy body disease, Parkinson's disease, Spinal muscular atrophy, Schizophrenia, Depression, Bipolar disease, Autism, Epilepsy, Frontotemporal dementia, Progressive Bulbar Palsy, Rett syndrome, Tourette syndrome, Neurofibromatosis, progressive muscular atrophy, hereditary spastic paraplegia, Pelizaeus-Merzbacher disease, Gaucher's disease, Spinocerebellar ataxia, Pagon Bird Detter syndrome; Friedreich's ataxia; Spinocerebellar ataxia, Digeorge syndrome, Dup15q syndrome, Doose Syndrome, Glut1 Deficiency Syndrome, CDKL5 Disorder, Frontal Lobe Epilepsy, Childhood Absence Epilepsy, Early Myoclonic Encephalopathy (EME), Lennox-Gastaut Syndrome (LGS), Ohtahara Syndrome, and Landau-Kleffner Syndrome,
b) said single-stranded antisense oligonucleotide is deliverable to the spleen, and wherein the medicament is for the treatment of a disease selected from the group consisting of cancer such as spleen cancer, an inflammatory disease and an immunodisorder,
c) said single-stranded antisense oligonucleotide is deliverable to T-cells, and wherein the medicament is for the treatment of a disease selected from the group consisting of cancer, an inflammatory disease and an infection disease, and/or
d) said single-stranded antisense oligonucleotide is deliverable to the liver, and wherein the medicament is for the treatment of a disease selected from the group consisting of liver cancer, cardiovascular diseases, coagulation cascade deficiencies, inflammatory diseases, metabolic disease and infections, e.g. wherein the liver diseases are selected from the group consisting of hepatocellular carcinoma, liver malignancies metastasized from primary cancers in other tissues, diabetes type 1, non-insulin dependent diabetes, insulin resistance, control of blood glucose levels, HDL/LDL cholesterol imbalance, atherosclerosis, dyslipidemias, familial combined hyperlipidaemia (FCHL), acquired hyperlipidaemia, statin-resistant hypercholesterolemia, cardiovascular disease, coronary artery disease (CAD), and coronary heart disease (CHD), non alcoholic steatohepatitis (NASH), non-alcoholic fatty liver disease, obesity, acute coronary syndrome (ACS), thrombosis, rare bleeding disorders, hepatitis B or C, cytomegalovirus infection, schistosomiasis infection and leptospirosis infection, malaria, fasciola infection, rheumatoid arthritis, liver fibrosis, cirrhosis, hepatic porphyria, acute intermittent porphyria (AIP), paroxysmal nocturnal hemoglobinuria (PNH), atypical hemolytic-uremic syndrome (aHUS), myasthenia gravis, neuromyelitis optica, alpha 1-antitrypsin deficiency, Cushing's Syndrome, and transthyretin-related hereditary amyloidosis.

18. A method for manufacturing an isolated milk extracellular vesicle carrying a single-stranded antisense oligonucleotide such as a single-stranded antisense oligonucleotide of 7 to 35 nucleotides with at least one backbone modification as defined in any one of the preceding claims, said method comprising the steps of
a) obtaining or providing bovine milk,
b) isolating milk extracellular vesicles from the milk obtained or provided in step (a), comprising
(b1) subjecting the bovine milk provided or obtained in step (a) to a series of sequential ultracentrifugations to obtain milk extracellular vesicles from the milk, and
(b2) purifying the bovine milk extracellular vesicles obtained in step (b1) by discontinuous iodoxanol density gradient purification,
and
c) encapsulating, by extrusion, a modified single stranded antisense oligonucleotide in the milk extracellular vesicles isolated in step (b), thereby manufacturing isolated milk extracellular vesicles carrying a modified single stranded antisense oligonucleotide.

19. The method of claim 18, wherein the hydrodynamic diameter of the isolated milk extracellular vesicle is in the range of 30 to 500 nm, wherein the isolated milk extracellular vesicle has a Zeta potential in the range of 0 to -50 mV, and/or wherein the isolated milk extracellular vesicle has a polydispersity index (PDI) in the range of 0 to 0.4.

20. An isolated milk extracellular vesicle carrying a single-stranded antisense oligonucleotide obtained by the process of claims 18 or 19.
